(19) Europäisches Patentamt
European Patent Office
Office européen des brevets

(11) **EP 4 150 386 B1**

(12) **EUROPEAN PATENT SPECIFICATION**

(45) Date of publication and mention
of the grant of the patent:
**27.11.2024 Bulletin 2024/48**

(21) Application number: **21719630.2**

(22) Date of filing: **19.04.2021**

(51) International Patent Classification (IPC):
***G02B 6/02*** *(2006.01)*

(52) Cooperative Patent Classification (CPC):
**G02B 6/02033; G02B 6/02042;** G02B 2006/0325;
G02B 2006/12069

(86) International application number:
**PCT/EP2021/060099**

(87) International publication number:
**WO 2021/228497 (18.11.2021 Gazette 2021/46)**

(54) **OPTICAL WAVEGUIDE AND METHOD OF FABRICATION THEREOF**

OPTISCHER WELLENLEITER UND VERFAHREN ZU SEINER HERSTELLUNG

GUIDE D'ONDES OPTIQUE ET SON PROCÉDÉ DE FABRICATION

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **13.05.2020 PCT/EP2020/063320**

(43) Date of publication of application:
**22.03.2023 Bulletin 2023/12**

(73) Proprietor: **Haute Ecole Arc
2000 Neuchâtel (CH)**

(72) Inventors:
 • **LLERA, Miguel
  2300 La Chaux-de-Fonds (CH)**
 • **FLAHAUT, Frédéric
  25500 Morteau (FR)**
 • **BERGERAT, Sylvain
  25210 Le Russey (FR)**

(74) Representative: **Bovard SA Neuchâtel
Rue des Beaux-Arts 8
2000 Neuchâtel (CH)**

(56) References cited:
**EP-A1- 0 727 054       WO-A1-2019/108862
CA-A1- 2 184 900        US-A- 4 909 597
US-A1- 2009 003 758     US-A1- 2016 281 267**

 • **ANDREAS LEBER ET AL: "Stretchable
  Thermoplastic Elastomer Optical Fibers for
  Sensing of Extreme Deformations", ADVANCED
  FUNCTIONAL MATERIALS, WILEY - V C H
  VERLAG GMBH & CO. KGAA, DE, vol. 29, no. 5,
  7 December 2018 (2018-12-07), pages n/a,
  XP072407234, ISSN: 1616-301X, DOI:
  10.1002/ADFM.201802629**

**Description**

Technical field of the invention

The present invention relates to the field of optical waveguides. The present invention more specifically relates to a method of fabrication of flexible optical waveguides such as optical fibers or flat waveguides that may be used advantageously in applications and devices wherein it is difficult to provide light to a distant target and in which the light path may be tiny, present short curvatures and/or complex shape. The invention relates to a method of fabrication of flexible optical waveguides, that are biocompatible and stretchable and are based on the use of elastomers. The invention proposes a solution to applications wherein breakage of an optical waveguide would have dramatic consequences.

Background of the invention

Optical waveguides constitute means to provide light to a distant target and may be deployed over long lengths and through narrow spaces and possibly harsh environments. Optical waveguides in the form of fiber optics, fiber bundles or flat waveguides have been developed for a wide range of applications such as telecom, industrial and medical applications. In the case of telecom applications, the focus has been put into processes that allow to provide extremely low absorption over very long lengths, and mainly in the infrared. Other applications such as industrial machines or medical application usually do not have this requirement but have other requirements such as their mechanical properties or also compatibility requirements which are mandatory in chemical, bio-chemical or medical environments. In the case of medical implants for example, the bio - compatibility is a main requirement. This bio - compatibility is mostly also linked to other requirements such as mechanical security requirements.

For example, glass optical fibers for human implants face many difficulties as biocompatibility or breakability that can be dramatic in such applications. Furthermore, implants fabrication often needs highly flexible optical waveguides because of the complex fabrication steps of the implants.

Most existing optical waveguides are based on glass or plastic fibers and are not suitable for some medical applications such as implants.

Several attempts have been made in the past to realize optical fibers having improved mechanical properties and/or their medical or biochemical compatibility. For example, polyurethane (PU) in optical fibers field has been widely used in the coating design and the fiber optic tubing for cable production.

Several solutions have been proposed to improve the mechanical properties of bent fibres. For example, documents JPS59111952 and WO2003091178A2 propose to use polyurethanes to increase the adhesivity of the coating on the glass fiber and to protect the fibre from the micro-bending effects. PU has also been used to reinforce optical fibers in order to increase their corrosion and weather resistance in aerial optical cables, as described in CN107589507. Also, the use of PU for better resistance of optical fibers for medical applications has been described in for example CN206431340. Also, the use of PU in complex coating structures in order to correct the defects on primary coatings has been proposed in US2013243948.

In the field of fiber-optic protection-tubing applications, which deals with the fabrication of optical cables, polymers such as PU has been proposed in CN203275734, solely as an additional component to increase the cable flexibility.

Other documents in the field of optical fibres propose the use of PU as unclad optical fibres. For example, US4915473A discloses a pressure sensor by using a PU fiber whose optical transmission is inversely proportional to the pressure applied on it. Also, US20080089088A1 describes to use an unclad PU fiber to produce a side scattering for lightning and decorative applications.

Flexible optical fibers for in-vivo use in the tissue of a living mammal is described in US4893897. The document US4893897 describes a fabrication process in which two materials, such as polystyrene and aliphatic PU, are used to produce the core and the cladding of the fiber. The process is based on the melting and co-extruding of the two materials to produce a fiber-like preform which is finally drawn to the required final optical fiber dimensions. A main constraint of the fabrication of US4893897 is that the cladding material must have a melt viscosity lower than the one for the core.

Another document JP 62269905 describes how to produce a flexible optical fiber by injecting a liquid PU resin onto a hollow flexible fiber and then by polymerizing this liquid resin with a UV light. The photocurable liquid resin is for example polyurethane poly (meth) acrylate alone but could also be a monovinyl compound such as alkyl (meth) acrylate or other materials. For the hollow fiber production, the materials used were polytetrafluoroethylene, ethylene-vinyl acetate copolymer, vinyl chloride resin, and other kind of materials. The hollow fiber must be extruded by using a concentric annular shaped die. Then the liquid resin is pushed on one side and sucked by using a vacuum pump on the other side of the hollow fiber. The polymerization of the liquid core is performed by UV light. The hollow fiber production generates very low-quality surfaces that leads to huge absorption effects and so to unacceptable optical transmissions.

Early use of optical silicone to produce optical fibers was described in the document JPS5447667 that describes a glass core covered with a silicone cladding layer. The use of silicone as a coating of glass optical fibers is described in several documents such as JPS6230152 and JPH01286939, CN108977069. Even with a silicone cladding these fibers remain unacceptable for several applications such as medical implants.

Polymer fibers such as silicone fibers have been described in for example US5237638A. The fabrication of such polymer fibers is realized by dipping an extruded core into a cladding solution and then by curing the cladding. Liquid silicone can be used to produce fluid light guides, as described in US5692088A. Such liquid silicone waveguides use a flexible tube with a specific film fixed at the internal surface to play the cladding role while the core is a liquid polymer as a fluid silicone. This technique can be used to produce liquid core flexible catheters for laser ablation, as described in US9700655B2. Silicone light guides use non-curable liquid silicone and the cladding is realized by a specific treatment on the internal tube surface and the guide sizes are on another order of magnitude. Such optical fibers are limited to cores that have a large lateral cross section and the production process is difficult to reproduce relative to the required optical properties. Also, these kinds of optical fibers are only used for light delivery systems where the core diameter is less important. For sensing applications, a single-mode operation is more suitable.

Highly stretchable optical fibers are described in US2012244143 and CN107907484. The document US2012244143 proposes the use of silk while the documents CN107907484 and US2016177002 describe the use of hydrogel. Approaches based on silk or hydrogels are not useful for different applications or configurations such as the use of them to interrogate optically resonating cavities such as Fabry-Perot cavities arranged at the end of a waveguide.

Realizing thin mechanical filaments intended for 3D printing solutions having specific properties for 3D printing devices have been described in the document US 2016/281267A1. But this document does not describe how to fabricate very thin optical fibers that must comprise a core and a cladding to assure to guide electromagnetic waves.

The following publication describes a stretchable thermoplastic Elastomer Optical Fiber of Extreme deformations: Andreas Leber et Al, ADVANCED FUNCTIONAL MATERIALS, WILEY-V CH VERLAG GMBH&CO, KGAA, DE, vol.29.nr.5, 7 December 2018 , XP072407234, ISSN:1616-301X, DOI: 1 002/ADFM.201802629. The problem of the optical fiber as described is that the used method relies on a method of extrusion and it would not be possible to realize optical fibers having a core that has a very small diameter, for example a core having a size of for example $10\mu m$ or $5\ \mu m$.

Furthermore, US4909597A discloses a method of fabrication of a polymer optical waveguide possessing a high degree of flexibility. More particularly, this polymer optical waveguide contains a thermoplastic aliphatic segmented polyurethane core and clad materials including polysiloxanes.

There is thus a need for improved waveguides, such as optical fibers because existing waveguides that are based on glass or plastic fibers are not suitable for a wide range of applications, such as implants or other medical applications.

Summary of the invention

The inventors of the present invention have found solutions to the above-discussed problems by providing a method of fabrication of optical waveguides, such as optical fibers, having a cladding made of a thermoplastic elastomer (TPE) such as a polyurethane. The fabrication process of the fibers and waveguides of the invention provides a wide range of advantages such as the decrease of the inherent complexity of optical waveguide production to a level where implant manufacturers could, at least partially, produce their own optical waveguides.

The invention is achieved by a method of fabrication of an optical waveguide according to any one of the independent claims 1, 3 and 4. Further embodiments are presented in dependent claims 2 and 5.

The optical waveguide realized by the method of the invention may be used in association with a chirurgical instrument during a chirurgical operation. In variants said use may be made for the tracking of the localisation of the position of said chirurgical instrument and/or the tracking of optical properties of tissues in the neighbourhood of the tip of said chirurgical instrument.

Brief description of the drawings

Figure 1 shows an optical waveguide realized by the method of the invention;

Figure 2 shows a longitudinal cross section of a waveguide realized by the method of the invention illustrating its optical acceptance angle and the angle of an outcoupled light beam;

Figure 3 shows a flat optical waveguide according to the method of fabrication of the invention;

Figure 4 shows a tapered optical fiber realized by the method of the invention;

Figure 5 shows a lateral cross-section of a fiber bundle comprising optical fibers realized by the method of the invention;

Figures 6-9 show exemplary cross sections of different types of optical fibers having at least two light guide cores imbedded in a polymer cladding realized by the method of the invention;

Figure 10 illustrates some steps of the fabrication, according to the invention, of an optical waveguide;

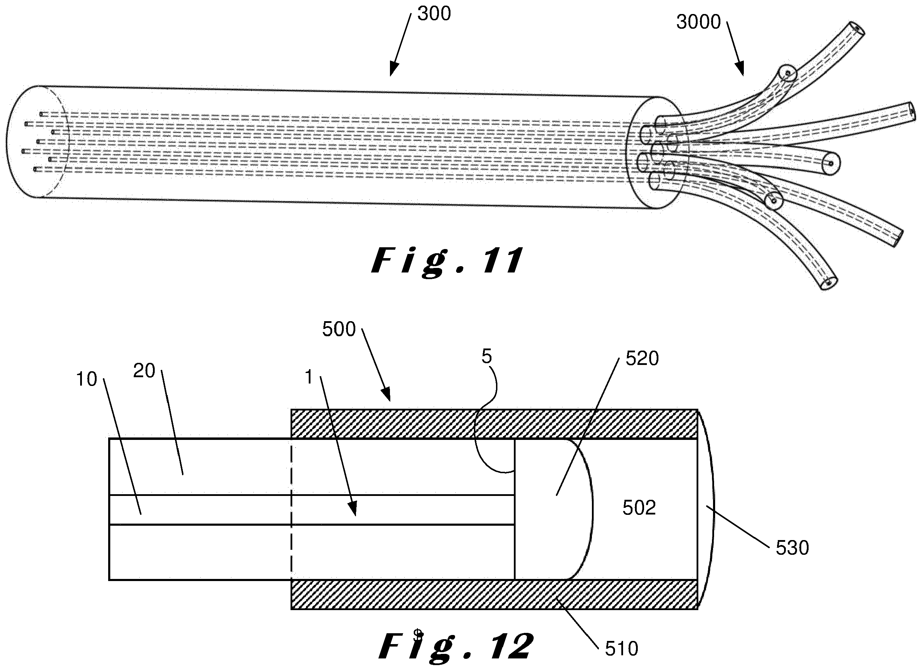

Figure 11 illustrates a hybrid preform that may be used to realize a Fan-In/Fan-out optical component realized by the method of the invention;

Figure 12 illustrates a sensor head comprising an optical cavity that comprises an outer membrane, said cavity being arranged by an outer tube on an optical fiber realized by the method of the invention;

Figure 13 illustrates an optical fiber realized by the method of the invention comprising a lateral incoupler grating and a lateral outcoupler grating;

Figure 14 illustrates a cochlear implant comprising an optical fiber realized by the method of the invention;

Figure 15 shows an example of a portion of a preform comprising two holders to make a fan-in/fan-out optical component realized by the method of the invention;

Figure 16 shows a portion of a preform comprising two holders to make a fan-in/fan-out optical component, the two holders comprise a plurality of wires to be removed after injection of a TPE polymer;

Figure 17 illustrates a preform after overmolding of a TPE polymer of the wires that are between the two holders of the arrangement of Fig. 16;

Figure 18 illustrates the demolding of a hybrid multicore fiber and fiber bundle preform after the injection of a TPU layer as cladding layer;

Figure 19 shows a hollow shaped preform made of TPU;

Figure 20 shows an end face of an optical fiber having a core and cladding made of two types of TPU and realized by the method of the invention;

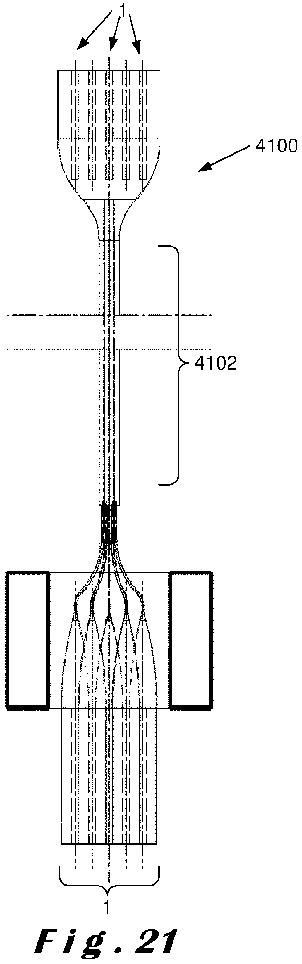

Figure 21 illustrates a multifiber Fan-in/Fan-out platform realized by using the mold of Figure 16 and Figure 17;

Figures 22-23 show experimental results obtained with an optical fiber realized by the method of the invention;

Figure 24 illustrates a far-field distribution of guided modes of a waveguide realized by the method of the invention that has been elongated at different percentages;

Figure 25-27 show complex -shaped cross sections of a preform realized by the method of the invention, realized by a 3D printing technique;

Figure 28 illustrates the realization, by 3D printing, of a full preform by depositing successive layers that comprise a core portion and an outer portion so as to form a full preform at the end of the 3D printing process.

Detailed description of the invention

The present invention will be described with respect to embodiments and with reference to the appended drawings, but the invention is not limited thereto. The drawings described are only schematic and are non-limiting. In the drawings, the size of some of the elements may be exaggerated and not drawn on scale for illustrative purposes. The dimensions and the relative dimensions do not correspond to actual reductions to the practice of the invention.
As used herein, the term "optical waveguide" - defined also as waveguide - used herein encompass all types of homogeneous or non - homogeneous and/or tapered optical waveguides such as monomode and multimode fibers but also monomode and multimode flat optical waveguides and also waveguide bundles that comprise a plurality of optical fibers or flat optical waveguides or a mix of them. Waveguides 1 have a longitudinal axis which is defined as a central virtual axis of the waveguide 1 defined in the direction of the guidance of an optical light beam 100 in the waveguide 1. Optical guidance may be performed by total internal reflection (TIR) or by using reflecting or diffracting layers or structures. In the case of monomode waveguides only one mode is guided through the core of the waveguide. Said virtual axis defines a Z-axis and two orthogonal axes X, Y or directions to said Z-axis. Lateral cross sections herein are cross section defined in a X-Y plane. Longitudinal cross sections are defined in a plane comprising said Z-axis.

The terms "cladding" and "core" are also defined as cladding and core layers and may be layers that do not have a circular cross section. This is valid as well for the core and cladding of the preform as described, as for the core and cladding realized optical waveguides.

The types of the optical waveguide 1 realized by the method of fabrication of the invention will be chosen according to the type of application or geometric constraints and the geometrical and working temperature requirements of the device wherein optical waveguide 1 is implemented and are typical, but not exclusively the following choices:

- single fibers: for transmitting intensity, polarisation and spectral information;
- fiber bundles: for transmitting images and illuminating light beams;
- multi-core optical waveguides such as multi-core optical fibers;
- flat waveguides: for transmitting intensity, polarisation and spectral information, as well as the transmission of images and illumination light beams;
- Fan-in/Fan-out optical devices.

The use and optical functioning of optical waveguides 1, defined also herein as waveguides, such as optical fibers 1 and optical fiber bundles 300 are well known to the skilled person in the field of guided optics and are not described further here. It is also known how to configure an optical fiber arrangement suited for illuminating an object and collecting reflected or transmitted light by such an object. The invention proposes a method of fabrication of optical waveguides such as optical fibers. As described further in detail, one of preferred fabrication methods includes a realisation of a thermoplastic elastomer (TPE), hollow shaped preform 204, a such as thermoplastic polyurethane (TPU) preform, that may be thermally drawn to obtain a highly flexible TPU capillary 2000. Waveguides 1 are fabricated by the method of the invention by either filling a hollow shaped preform 204 with a polymer and pulling them to obtain a thin waveguide 1, or by first realizing a solid capillary and filling it with a liquid polymer. Different ways of the curing of the liquid polymer of the core 10 are described further. The liquid to be used to form the core 10 of the waveguide 1 is silicone, or siloxane.

The invention proposes a method of fabrication of a new biocompatible and highly stretchable optical waveguides 1 by using low-cost, biocompatible and optically transparent materials with a much more reduced multimode behaviour. The invention relates to the use of a thermoplastic elastomer (TPE) for the cladding of the optical waveguide 1 and the preform to make it. The cladding 20 of the waveguide 1, realized by the method of fabrication of the invention, is composed at least partially of a thermoplastic elastomer (TPE), preferably a TPU elastomer. The cladding is made from a hollow preform 204 composed at least partially, or entirely, of thermoplastic polyurethane. As described further the core 10 of the optical waveguide is another type of TPE than the one used for the cladding or is a liquid silicone that is polymerized in a realized capillary shaped cladding.

It is understood that the optical waveguides 1 realized by the method of the invention may be arranged in a wide variety of forms and geometries or may be arranged in any configuration in a medical device 2. An optical waveguide 1, realized by the method of fabrication of the invention, may comprise a core layer 10, defining a longitudinal axis Z, and a cladding layer 20 surrounding said core layer 10 , said optical waveguide 1 having an incoupling surface 31 for incoupling a light beam 110 into said core layer 10 and an outcoupling surface 51 for outcoupling light 120 out of said core layer 10, said core layer 10 and said cladding layer 20 being configured to transmit along said longitudinal axis Z a guided light beam 100, through said core layer 10 and from said incoupling surface 31 to said outcoupling surface 51, said guided light beam 100 having a wavelength greater than 180nm. In all the embodiments of the invention the cladding layer of the optical waveguide 1 is composed, at least partially, of thermoplastic elastomer (TPE). which is a thermoplastic rubber. TPE is a thermoplastic elastomer and is part of a class of copolymers or a physical mixture of polymers, usually a plastic and a rubber, that consists of materials with both thermoplastic and elastomeric properties. Thermoplastics components are relatively easy to manufacture, for example by injection moulding Thermoplastic elastomers present both the advantages of rubbery materials and plastic materials. The benefit of using a thermoplastic elastomer, such as TPU, as a material for at least the cladding of the optical waveguide 1 is the surprising ability to stretch to moderate elongations and return to its near original shape creating a longer life and better physical range than other material. The principal difference between thermoset elastomers and thermoplastic elastomers, such as TPU, is the type of crosslinking bond in their structures. In fact, the crosslinking property is a critical structural factor which imparts the high elastic properties of the optical waveguide realized by the method of fabrication of the invention. TPE is well known and not further described here.

There are six generic classes of commercial TPEs (designations according to the ISO norm 18064):

- styrenic block copolymers, TPS (TPE-s);

- thermoplastic polyolefin elastomers, TPO (TPE-o);

- thermoplastic Vulcanizates, TPV (TPE-v or TPV);

- thermoplastic polyurethanes, TPU (TPU);

- thermoplastic copolyester, TPC (TPE-E);

- thermoplastic polyamides, TPA (TPE-A);

- not classified thermoplastic elastomers, TPZ.

Herein, the cladding is at least partially composed of a TPE but other different TPE materials may be combined for realizing the cladding layer. In a preferred embodiment the entire cladding layer is made of TPE, preferably entirely made of TPU which is particularly well suited for certain applications such as medical devices.

All mentioned TPE may be used to realize the preform 200 and the optical waveguide 1 realized by the method of fabrication of the invention. TPU is the preferred choice among these TPE materials. The reason is that it has the best combination of thermoplastics and rubbers (thermosets) for the desired properties of the optical waveguide 1 realized by the method of fabrication of the invention, as described further in detail. The cladding layer 20 may be a homogenous layer but may also be made of at least two sections that have a different layer composition. The cladding may be made by at least one layer that is made of a homogenous TPE layer but may also be made by a non-homogenous TPE layer. In variants the TPE layer may comprise at least to one of its sides at least another layer made of TPE. This may be used to realize for example a gradient index optical waveguide 1 comprising a plurality of TPE layers with different refractive indexes. Said another TPE layer may be a non-homogenous layer which may have a diameter that varies along the length of the cladding layer. As further described such a non-homogeneous layer may be an external layer that has a mechanical function and which is incorporated to the cladding of the waveguide and may be used for improved mechanical adherence or clipping or gripping or sliding into a device.

The core layer 10 is made of a first material having a first index of refraction n1, and the cladding layer 20 is made of at least one layer composed at least partially of TPE, for example TPU, having a second index of refraction n2 that is smaller than said first index of refraction n1.

In a specific case the optical waveguide 1is a capillary made of at least one cladding layer that comprises an internal reflection layer and the core layer 10 may be air or vacuum or may be a liquid, such as oil. In such cases the waveguide comprises a closure at each end of the capillary structure.

The values of refractive indexes of the core and/or cladding layers 10I, 20 can cover a broad range for both materials for example n2 may be between 1.49 and 1.57 and n1 may be between 1.52 and 1.60. It is understood that, in a preferred embodiment the core 10 and cladding 20 of the optical waveguide may be uniform layers that have a uniform refractive index n1, respectively n2. The cladding is in contact with the core. As well the cladding 20 as the core 10 may comprise different layers that are in contact. The inner layer of the cladding 20 is in contact with the outer layer of the core 10 to ensure total reflection. The core 10 and cladding 20 may be composed of a succession of layers that have different discrete refractive indexes. The core and/or cladding may have a gradient index that varies continuously over the diameter of the core 10 and/or cladding 20, which may be realized by doping the deposited polymers during their manufacturing. Anyhow, the refractive index of the outermost layer of the core 10 has a refractive index that is higher than the refractive index of the innermost layer of the cladding 20, said innermost and outermost layers being in contact.

The limitation of the useful length of the proposed optical waveguide 1 may be the penetration length of the core material, such as in the case of the use of a liquid silicone or another TPU composition as the core material to be introduced into a TPE capillary as further described in the method section. TPU is a preferred choice among the TPEs as it possesses rubber-like elasticity, high tear and abrasion resistance, high elongation at break as well as excellent thermal stability. In addition to this, TPU is also resistant to oils, greases, and a variety of solvents. TPU is also the firmest of TPE and may be printed by 3D techniques as further described. The use of TPU allows to fabricate complex internal and external structures of the optical waveguide 1. Additionally, TPU may be provided in a huge range of colours, has low shrinkage and is excellent for applications wherein vibration damping, and impact resistance are important. Waveguides 1 made in TPU are particularly suited for medical applications because of these properties. For applications wherein such stringent requirements are not mandatory, the cladding and possibly also the core of the waveguide 1 may be made by another type of TPE. Different fabrication methods are possible that are described further, and each variant of other method allows to achieve different geometries and different lengths in function of the application and so the required intensity throughput or the required intensity variations. Nevertheless, a typical optical waveguide 1 is an optical fiber having a fiber length of around 10 cm, for example in the case of medical implants, but it may longer. For example, a length of 10 cm would be enough for an organ-scale distance that is over 10 cm for humans [Ref. 5].

In preferred embodiments, the lineal loss of the optical waveguide 1 does not exceed 0.5 - 1 dB/cm, which ensures an overall loss of maximum 10 - 20 dB for a round trip on a 10 cm long waveguide. The bending losses are of high importance in applications as cochlear implants [3] where the waveguide 1 must be arranged on tight radii as we can found inside a cochlea (1-2 mm at the far end). However, these radii are progressive and the waveguide 1 may be placed through places having very short bending radii only over a few millimetres. In order to provide some margin on the final retrieved signal coming from a resonating cavity at a fiber tip for example, it is essential that the waveguide 1 has less than 5 dB loss on a round trip base for a bending radius of about 5 mm over a fiber length of 30 mm that corresponds to an average cochlear length. Obviously, this constraint is driven by the waveguide attenuation itself. If the fiber attenuation is much lower than the 0.5 -1 dB/cm specified above, then the margin available for the bending loss will be higher.

Typical attenuation values are 0.79dB/cm at 1550 nm and 0.46 dB/cm at 1300 nm. At 633 nm, attenuation values are lower 0.79dB/cm at 1550 nm and 0.46 dB/cm at 1300 nm So, optical waveguide lengths of more than 2 m may be used. Experimental data have shown that the optical waveguide 1 realized by the method of the invention has a lower attenuation in the visible part of the spectrum than in the infrared part of the spectrum.

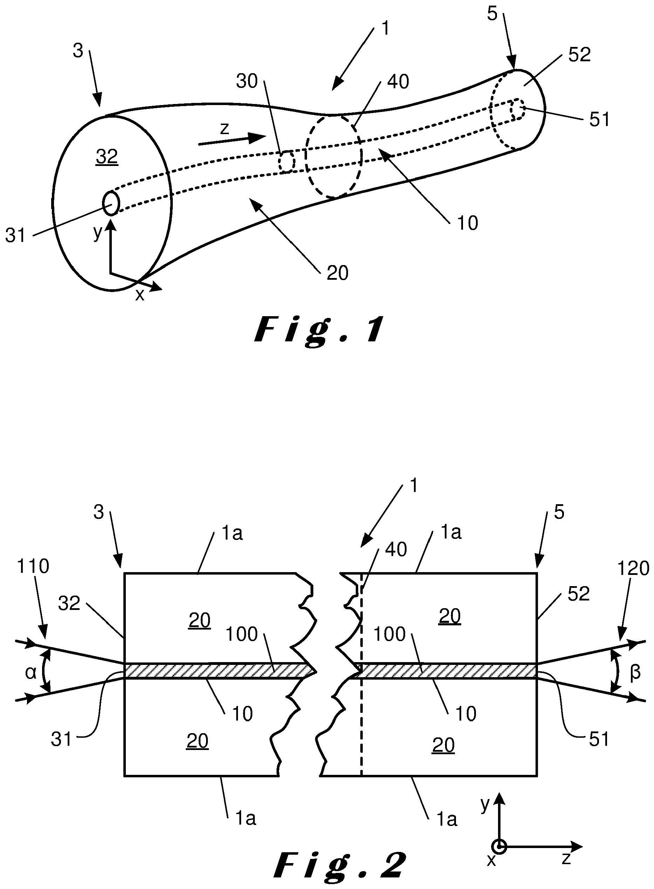

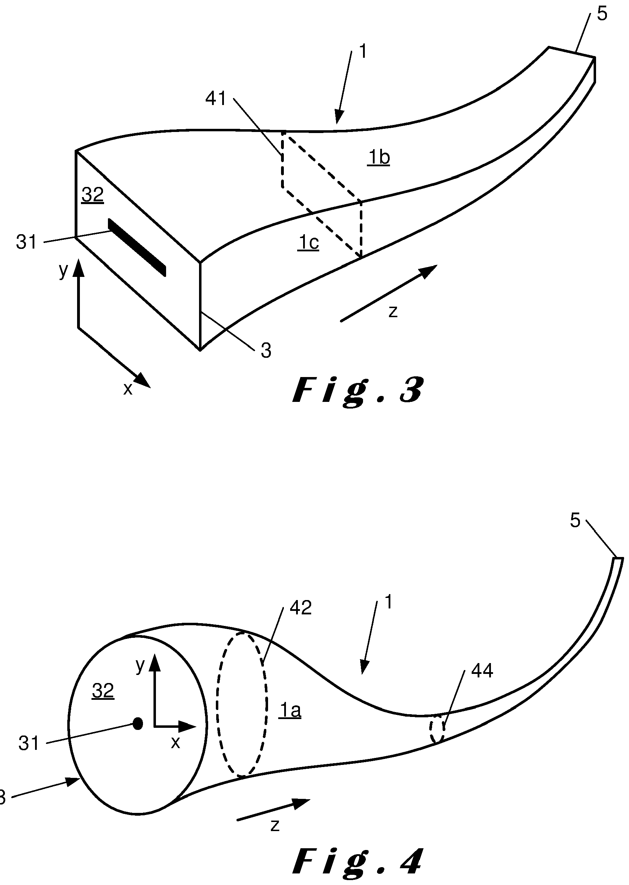

In a preferred embodiment, the optical waveguide 1 is a monomode or a multimode fiber, as illustrated in Fig.1. The lateral cross section 30 of the core 10 and the cross section 40 of the cladding 20 may be uniform over the length of the waveguide 1, but may also vary as illustrated in Fig.4.

In an embodiment, illustrated in Fig.3 the optical waveguide 1 has a first lateral side 1c having a first width W1 and a second side 1b having a second width W2 that is larger than said first width W1, said widths W1, W2 being defined in any lateral cross section, defined in an plane X-Y orthogonal to said longitudinal axis Z. Fig.3 shows a flat optical waveguide 1 having a rectangular shaped cross section, but other cross sections may also be possible, such as elliptical shaped cross sections, or trapezium shaped cross-sections.

In an embodiment, illustrated in Fig.4, the optical waveguide 1 is a tapered waveguide 1, having a tapered form in at least one plane comprising said longitudinal axis Z. Fig.4 illustrates a varying shape and/or dimension of lateral cross sections 42, 44.

In an embodiment, said core layer 10 is made of a polymer. This polymer may be silicone.

In variants the core layer may be a liquid. This may be realized by providing a capillary that has a very small core diameter so that the liquid remains trapped inside the optical waveguide 1. In a variant the input and output areas of a waveguide, realized by the method of fabrication of the invention and having a liquid core, may have a window to close of the liquid core so that the liquid remains inside the waveguide 1.

In an embodiment a reflecting layer may arranged between said core layer 10 and said cladding layer 20, said reflecting layer being arranged to provide inside said core layer 10, total reflection and guidance of incoupled guided light into said core layer 20. Said reflecting layer may be a metallic layer or a dielectric layer or a combination of them.

In an embodiment the optical waveguide 1 is an optical fiber wherein said core layer 10 and said cladding layer 20 are configured to guide a number of modes less than 100, preferably less than 20, more preferably less than 5. In an embodiment the optical waveguide 1 is a monomode fiber.

In an embodiment the optical waveguide 1 is configured to guide less than 100 modes, preferably less than 20 modes, more preferably less than 5 modes, defined in at least one longitudinal plane X-Z, Y-Z.

In an embodiment the optical waveguide 1 is a tapered optical waveguide having at least two different cross sections 42, 44.

The optical waveguide 1 has an optical transmission T0, defined as the ratio I2/I1 of the intensity I2 of the outcoupled light 120 to the intensity I1. In an embodiment the optical waveguide 1 has a practical length smaller than 2m, preferably smaller than 0.5m, more preferably smaller than 0.25m. and the intensity I2 of the outcoupled light 120 may be greater than 10%, preferably greater than 30% than the intensity T0 of the incoupled light 110, for incoupled light having wavelengths between 180nm and 25 $\mu$m.

In an embodiment a useful length of the optical waveguide 1 has an optical transmission that is greater than 80%, preferably greater than 90% for incoupled light having wavelengths between 300nm and 5 $\mu$m, preferable between 350nm and 2$\mu$m, even more preferably between 400nm and 700nm. In the case of medical implants for example. said useful length is typically 10-20cm.

In an embodiment the optical waveguide 1 can be elastically stretched up to at least 10% of its length L and so that, after having been stretched, the optical transmission T2 remains at least 50%, preferably at least 70%, even more preferable at least 90% of the transmission T0 of the optical waveguide 1 before being stretched.

One of the essential features of the optical waveguide 1 is that it may be stretched while maintaining substantially its optical guidance properties. In the case of a hollow core waveguide 1 made of TPU the core 10 is air or vacuum, and an elongation of 600% is possible before breakage of the waveguide 1. In the case of an optical waveguide 1 having a core 10 made of silicone the possible elongation before rupture may be similar depending on the adherence properties of the core layer 10 with the cladding layer 20. The rupture limit may also depend on the elongation properties of the core layer because, depending on the chosen core material layer, the core layer may be damaged or ruptured before the damaging of the cladding layer. Typical silicone core layers may have an elongation of up to 50% before rupture.

In an embodiment an adherence or an antifriction layer may be provided at the inner surface of said capillary 2000 before introducing said liquid core material. This provides ways to improve the breakage limit or possible mechanical damages to the waveguide 1 for example in situations of small curvature radii and/or high traction forces.

In an advantageous embodiment the optical waveguide 1 may be made, at least partially, electrically conductive, which is possible by providing an electrical conducting TPU material for the preform and so for the core and/or cladding of the optical waveguide. As described further, the 3D printing technique may also allow to imbed in the full or hollow preforms 200, 204, and thus the optical waveguide 1, at least one electrical and conductive wire which may be very useful in some medical devices. In variants the preforms 200, 204 may be doped preforms or preforms comprising a powders, such as an electrical conductive powder or substance. This may be useful to provide optical waveguides 1 that may be partially electrically or thermally conductive. In variants, only the cladding 20 of the optical waveguide 1 may be made electrically or thermally conductive.

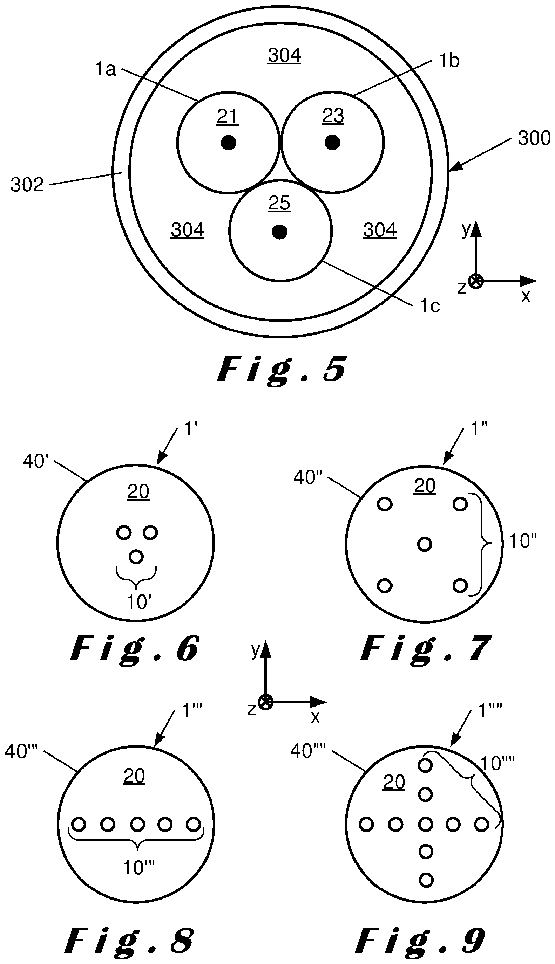

Fig 5 illustrates an optical waveguide bundle 300 comprising at least three optical waveguides 1a, 1b, 1c comprising fibers realized according to the method of fabrication of the invention. In a variant 7 optical fibers 1 may be arranged into such a fiber bundle 300 that comprises an outer mantle 302 and an inner filling material 304.

In embodiments illustrated in Figs.6 -9 an optical waveguide 1', 1", 1''', 1'''' may comprise a plurality of core layers 10', 10", 10''', 10''''.

In embodiments the cladding layer may comprise at least two layers, of which at least one layer comprises a TPU polymer or is made entirely of a TPU polymer. In embodiments the cladding layer may comprise at least five layers, allowing to provide for example a gradient index waveguide. In variants, the core 10 may also be made of at least two different layers, both may be a TPU layer or another type of TPE layer.

In embodiments the optical waveguide (1) may be a polarization maintaining waveguide (1). This may be realized by incorporating polarisation substances in the preform, possibly added by 3D printing as described herein.

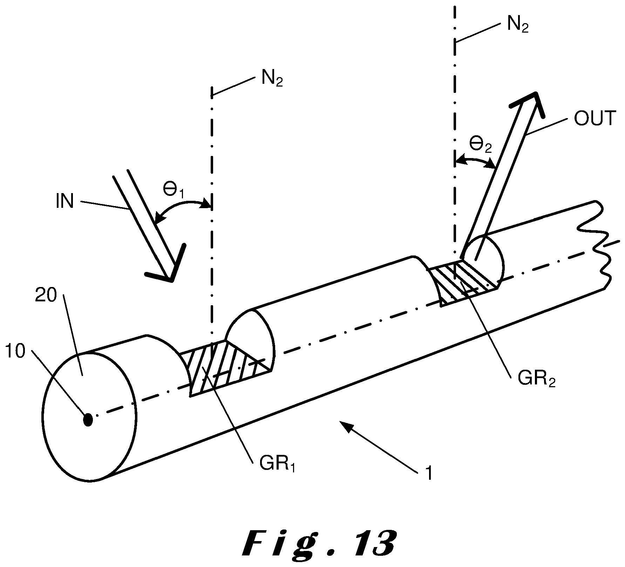

It is understood that the optical waveguide 1, realized by the method of fabrication of the invention, is not limited to only a waveguide 1 comprising a core layer 10 and a cladding layer 20. As well the core layer 10 and/or the cladding layer 20 may comprise structured portions that have an optical function. A typical optical structure is a diffraction grating that may be a local diffraction grating or a distributed grating, as illustrated in Fig. 13. Also, hologram-type structures or layers may be arranged into or on said optical waveguide 1.

In advantageous embodiments, at least a portion of said waveguide 1 is arranged according to a resonant waveguide grating (RWG). RWG's are described in for example:

- A.Sharon et al.:"Resonating grating-waveguide structures for visible and near-infrared radiation": J.Opt.Soc.Am" vol. 14, nr.11, pp.2985-2993, 1997.

RWG's are made by using a multilayer configuration and combine subwavelength gratings and a thin waveguide. A resonance occurs when incident light is diffracted by a grating and matches a mode of the waveguide. As most of the spectrum of incoupled light does not couple into the waveguide, strong spectral effects are provided in reflection and/or transmission. This to the fact that RWG's are corrugated waveguides and behave as a waveguide-grating. The use of RWG in indicia allows to provide unique optical effects that are extremely difficult to identify and to duplicate. RWG's are generally designed to have spatial periodicity shorter than the wavelength they operate with and are therefore called "subwavelength" structures or subwavelength devices. Eventually they have periodicities closed to the wavelength they are operating with and just above it. Quite often, the periods are significantly smaller than the free-space wavelength they are working with, for example a third of it. Because of their small periodicity, they do not allow various diffractive orders, which distinguishes them from much simpler diffractive optical elements (DOE).

Using RWG allows to provide unique incoupling and outcoupling optical effects, for example by providing a high incoupling and/or outcoupling efficiency or to incouple and outcouple polarized light beams more efficiently or with predetermined angles which would not be possible by using ordinary diffraction gratings such as binary diffraction gratings. RWG may be realized by embossing techniques allowing to provide cheap waveguide that have very efficient light coupling efficiencies that may depend, according to their design, particularly on specific predetermined wavelengths. In variants that are not illustrated in figures at least one of the lateral surfaces of the waveguide 1 is arranged, continuously or discontinuously, over at least 50% of its entire length, as an incoupling surface and/or an outcoupling surface. Said incoupling surface and/or an outcoupling surface may be configured as a RWG.

At least one optical waveguide 1 as described herein, may be used in optical systems or sensors. In an example, a resonating cavity is arranged as a tip of the optical waveguide 1 of the invention. As a light source low-cost telecom-grade LEDs may be used to interrogate the resonating cavity. In such devices a useful fringe visibility is required in cavity lengths of around 200 - 300 $\mu$m.

The optical waveguide as described herein may be used in an optical waveguide sensor that comprises a resonating cavity 520, arranged in a tip fixed to the output end of the optical waveguide deformable diaphragm 530. The cavity 520 may have another function than a resonating effect, for example the cavity may provide, through the deformation of the membrane, a varying light intensity of the light beam that is sent back into the fiber 1. In embodiments, cavities 520 may be filled with air or a liquid, such as oil.

The optical waveguide realized by the method of fabrication of the invention may be used in a pressure sensor. The pressure sensor may rely on the bending or elongation effects on the transmitted intensities of the optical waveguide as described in the experimental section further.

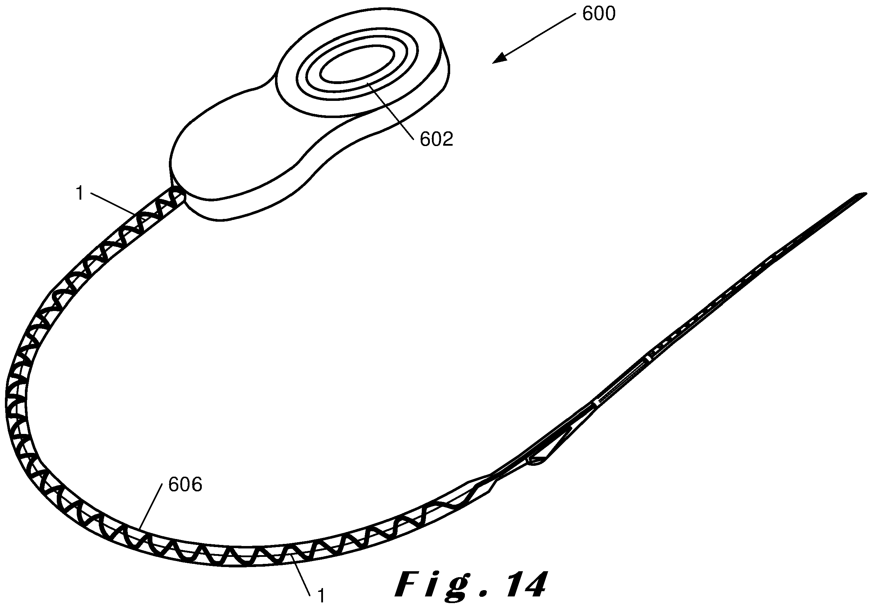

The optical waveguide realized by the method of fabrication of the invention may be used in a medical device is an implant to be used in cochleas. Fig.14 illustrates a cochlea implant 600 that comprises a central portion 606 to be inserted into the ear of a human being. A mechanical guidance structure 606 is arranged to said central portion and comprises at least one optical waveguide 1 according to the invention.

The medical device may comprise at least one optical waveguide 1, realized by the method of fabrication of the invention, to provide a UV-light beam to a predetermined location, for example to disinfect a location in a living body. It is generally understood that at least one extremity of the optical waveguide 1 may have a shape so that it may be used for an optical function such as the deviation or focusing or diverging of an incoming or outcoupled light beam. Said shape may be realized during the fabrication process of the waveguide 1, for example by heating the extremity so that a rounded shape is provided to an end of the waveguide.

The waveguide 1, realized by the method of fabrication of the invention, may be used for optogenetics described in Ref. 18.

The waveguide 1, realized by the method of fabrication of the invention, may also be used to track in real-time surgical instruments, for example to give information of the localisation of the tips of the instruments or to monitor optical information at the tip of the optical waveguide at the place of a surgical intervention. A surgical instrument may comprise the optical waveguide realized by the method of the invention.

The invention relates to the fabrication of an optical waveguide 1 as described in steps A, B, C of claim 1.

It is understood that the refractive indices of the core 10 and the cladding 20 of the optical waveguide 1 are, after the said reduction step C, within an error of 10%, substantially the same refractive index than the refractive indices of the core part respectively the wall of the hollow shaped preform 204.

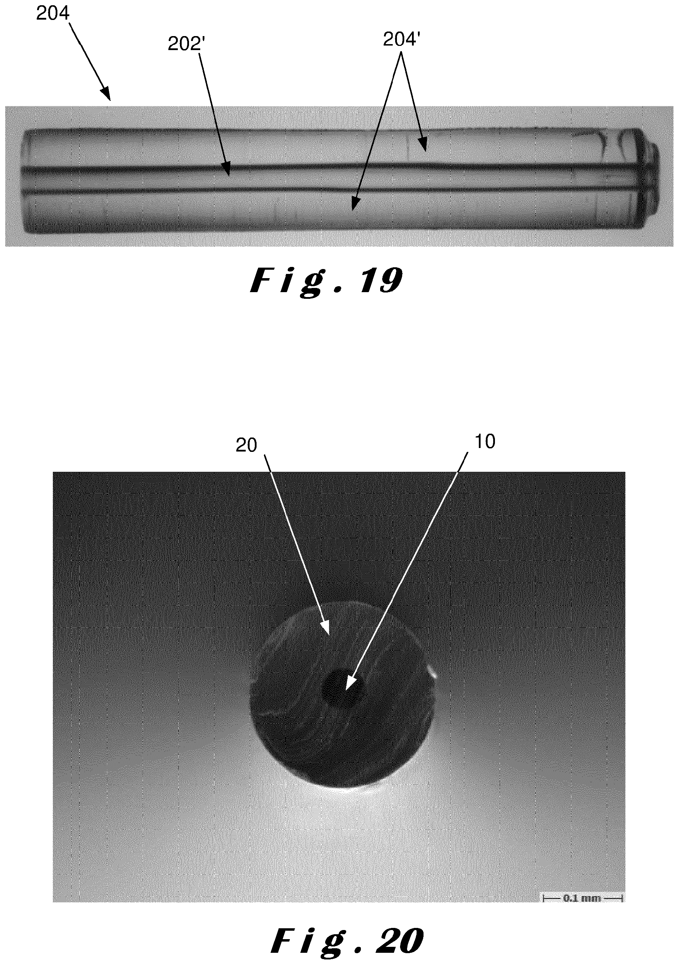

In an embodiment said preform 200 in step A is made by a first injection in a mould, typically a cylindrical-shaped mould. This mould is preferably a metallic or ceramic mould. The role of the cylindrical insert is to provide a preform having a central hole 202, defined also as central aperture. Fig. 19 shows an example of a realized TPU preform 200 that comprises an outer part 204, also defined as the wall of the preform, and a central aperture 202 that is to be filled by material that constitutes the core 10 of the optical waveguide when drawn from the preform 200. Said outer part 204' forms, when drawn, the cladding 20 of the optical waveguide and the inner part 202 forms, when drawn, the core 10 of the optical waveguide 1. In a variant a hollow preform may also be first drawn to a certain length and provide a TPE capillary to be filled with core material and polymerised.

In an advantageous embodiment said cylindrical preform 204 in step A is made in step A by a 3D printing technique.

In an embodiment the filing step B is made after step A has been executed, for example by an injection technique.

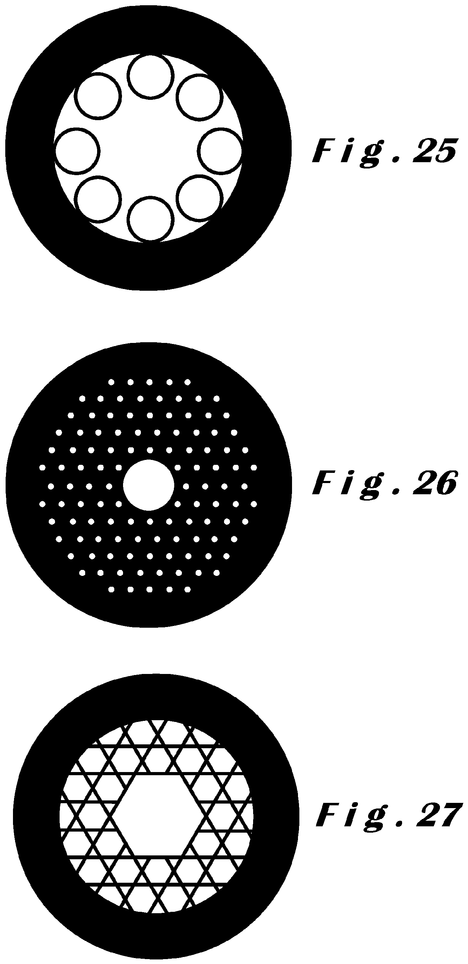

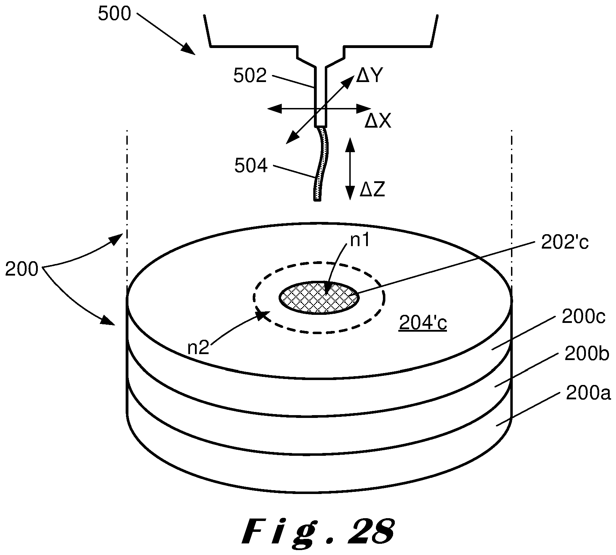

In an advantageous example, not forming part of the claimed invention, the step A and B is performed simultaneously by a 3D printing technique. By using such a 3D printing technique, the core and the cladding layer of the preform are built by adding successive layers. In each such layer a different PME elastomer is printed inside a layer of TPU, preferably a ring-shaped layer. TPU is a preferred choice of TPEs as it provides a layer to layer adhesion that may be printed more strongly and durable than other TPEs. The applications of the optical waveguide 1 realized by the method of fabrication of the invention focus on speciality waveguides such as needed for medical devices. Therefore, a slow printing speed can be allowed to realize the preform which may have a very complex shape. As the required lengths for the applications of the waveguide realized by the method of fabrication invention are small lengths it is acceptable that the time to process the preform 200 may be much longer than what is usually required for telecom preforms. Out of 1 preform thousands of short lengths of optical waveguides may be provided. Fig.28 illustrates a method of formation of a completely filled preform 200 by a 3D machine 500, not forming part of the claimed invention. In the example of execution of Fig.28 a single nozzle 502 provides a stream 504 of polymer. In the example of fig.28 the core part 202 and the outer part 204' of the preform 200 are realized by 3D printing of successive layers 200a-200c. Fig.28 only shows the first 3 layers 200a-200c that have been formed during a 3D printing operation. Each of the successive layers 200a-200c comprises a central portion 202a-202c and an outer portion 204'a-204'c , the central portion 202a-202c having, at least to its side of said outer portion, a first index of refraction n, said outer portion 204'a-204'c being made, at least to its side of said inner portion 202a-202c , at least partially of a thermoplastic elastomer (TPE) having a second index of refraction n2 being smaller than said first index of refraction n1. In examples, not forming part of the claimed invention, several variants to the 3D printing process may be conceived. For example, more than one central portion may be deposited during each layer step. The advantage of using a 3D printing as described here is to allow to provide complex shaped core parts and/or outer parts of the preform 200, such as illustrated in the example of Figs.25-27.

In embodiments, the material of the core part 202 of the preform 200 may be thermoplastic elastomer (TPE) layer chosen among one of: a styrenic block copolymer (TPE-s), thermoplastic polyolefin elastomers (TPE-o), a thermoplastic Vulcanizate (TPE-v, TPV), a thermoplastic polyurethane (TPU), a thermoplastic copolyester (TPE-E), a thermoplastic polyamide (TPE-A) or not classified thermoplastic elastomers,(TPZ), said thermoplastics being defined according to the ISO norm 18064.

The advantages and more details on the use of TPE, preferably TPU, for the preform and optical waveguides 1 realized by the method of fabrication of the invention are now described in more detail.

Elastomers are based on relatively long polymeric chains having a high degree of mobility and flexibility. Those chains are linked to a network structure that prevents them to flow from each other when an external stress is applied. That configuration resides on a dual-phase material where one phase is hard and solid whilst the other one is an elastomer. The elastomeric phase will offer the elasticity of the material and the solid phase will bring the physical crosslinks keeping the strength of the material. When processing such material, two different glass transition temperatures are involved: one for the elastomeric polymer, Te, and another one to the hard phase, Th, where Th > Te. Basically, the material will need to be used between those two temperatures as for temperatures T < Te, both phases are hard and brittle and for T > Th, the material will start to be a viscous fluid. That is of importance for the production of an optical waveguides, such as optical fibers, due to the fact that weakly guiding fibers need, as a basic configuration, a step-index refractive index and hence two different polymers, such as tow different TPUs. Care needs to be taken in the choice of the two TPU materials needed to produce the step-index profile. The temperatures are usually well below the 0°C temperature and are of less concern in the choice. However, Th will have a direct influence in the fiber drawing as one of the two polymers, for example two different TPUs, will start to melt at a lower temperature than the second one. Furthermore, the heating system, if not correctly designed, will generate a non-homogenic radial heat distribution into the preform enhancing the Te difference effect.

In an exemplary embodiment two TPUs are used to produce an optical fiber: i.e. BASF Elastollan 1185A and Ellastollan 1185 A10W having refractive indexes, nd, of 1.505 and 1.553, respectively. The processing temperatures for both TPUs are relatively similar and then offer an eased selection of the right drawing temperature.

In a typical execution, the preforms 200, 204 may be realized in a two-step injection moulding process using for example an Arburg Allrounder 170S injection machine. Said first injection is performed in a cylindrical mould having dimensions of 15 mm in diameter for 100 mm in length with a cylindrical insert in the middle along the cylinder axis. In a preferred embodiment the first step preform generates the cladding part of the fiber by using the Elastollan 1185A TPU. A second injection, using the Elastollan 1185 A10W, may be realized in order to fill the hole and produce the core part of the preform.

In embodiments the extruder of the 3D printer has a fully enclosed PTFE lined filament guide to prevent the filament from buckling during the printing process.

In variants the nozzle has an interior diameter of 0.25 mm, possibly as small as 0.1mm. In order to provide a smooth PTE filament, preferably a PTU filament, an internal pressure system may be provided.

In order to provide precise structures in the model, the 3D system may be provided with a X and Y and Z displacement mechanism of the nozzle or nozzles, having a precision of less than $10\mu m$, possibly less than $5\mu m$, possible having a precision of less than $2\mu m$. In advantageous variants the volume deposition speed may be greater than 10 $mm^3$/s, possibly greater than 24 $mm^3$/s. Compared to rigid thermoplastics at nominal nozzle temperatures, TPU has a much lower viscosity. This allows for high intermolecular diffusion or healing during the FDM process. So, TPU is a better choice for realizing optical waveguide preforms than other polymers.

In embodiments, the filled or hollow shaped preforms 200, 204 may be made with at least one layer of TPE, preferably TPU, to which at least another layer is arranged. Such additional layer may be realized to the inner wall of the cylindrical aperture of the TPE layer or to its outside surface.

The preforms 200, 204 made at least partially of TPE, preferably TPU, may comprise a plurality of layers having a predetermined order of refractive indices so that for example a gradient index layer is provided to the core layer 10 of the optical fiber 1 when it has been realized by elongating the preform 200. Providing optical waveguides having a gradient index profile around its core 10 has several advantages relative to the propagation characteristics of a guided light beam. The gradient index profile may be adapted to control the type and number of guided modes in the optical waveguide 1.

In an advantageous embodiment the inner surface of the TPE-based preforms 200, 204, preferably a preform made in TPU, is polished to improve the smoothness of its surface in order to reduce optical losses due to the interface of the core and cladding of the produced optical fibers as commented further in detail in the experimental section.

3D printing of a full or hollow preform 200, 204 that is made at least partially of TPE, preferably TPU, allows to provide conventional step-index optical fibres and dopant distributions that would be difficult, if not impossible to achieve with standard injection techniques to fabricate optical waveguide preforms. The reason is that the geometry of each layer that is created with a 3D print process can be adapted. In a first aspect there is a benefit for realizing micro-and nanostructured optical waveguides 1 such as optical fibres. The fabrication of these fibres is limited to methods that are often only suitable for specific materials. In a second aspect the outer and inner cross sections may have a shape that is predetermined. For example, the cross section may be square or hexagonal over the entire length of the preform.

Additionally, the shape of the cross section may vary over a predetermined length of the preform. For example, the preform may have a hexagonal cross section over 80% of its length and the two extreme parts may have another cross-section shape, for example a circular shape. In variants, the preform may be made, over a first length, a first type of TPE, such as a TPU elastomer, and over a second length be made of another type of polymer, for example another type of TPE or TPU. This is made possible easily by a 3D printing technique.

In embodiments, the preform 200, 204 and so the optical waveguide 1 may have a Y-shape or any other shape presenting more than 2 branches, to provide optical waveguides 1 that may have N incoupling branches and M outcoupling branches, M being equal or greater than N. For example, a TPU based optical waveguide may have 2 incoupling sections and 4 outcoupling sections. This would be extremely difficult, if not impossible, to realize with injection techniques, to the contrary of the 3D printing technique proposed by the invention.

In other embodiments, the cladding 20 may be formed around at least one solid element, such as a solid axis, so that the waveguide is firmly attached to that element without having to glue or fix it. This avoid wrapping or fix a waveguide 1 to such an element, which may be a component of a medical device, allowing to reduce the costs.

Realizing the preform with a 3D printing technique is particularly interesting in the frame of the present invention because the optical waveguides 1 can have a particular outer and/or inner shape that allows the waveguide to be easily adapted into or on a chirurgical device. For example, the filled preform , i.e. having a cladding and a filled core, may be realized so that there is at least one longitudinal aperture in for example the cladding, allowing to fit the optical waveguide 1 onto a very thin guidance wire. In variants, the preform, and thus the final optical waveguide, may have an undulated surface in their length and/or their width.

In another aspect, by realizing a preform with a 3D printing technique one may easily make waveguides that have a donut-shaped cross section or a halter shape that comprises 2 cores. In variants hexagonally packed preforms and fibers may be realized with the 3D process. In other variants the preform and so also the manufactured optical waveguide thereof is made, at least partially, of a photonic crystal.

In yet another aspect, 3D printing of the TPE cladding, preferably a TPU cladding, and/or a filled preform realized entirely with a TPU elastomer allows to add mechanical forms and features to the outside of the formed optical waveguide, which has an interest in the mounting and fixing in narrow chirurgical instruments. For example, during the 3D printing lateral structures may be added to the preform, and thus the later realized optical waveguide, said lateral structures may be realized in for example a harder polymer. By using a 3D printing technique, at least two deposited layers may have different compositions. It would be particularly difficult to realize the above-mentioned shapes by realizing a TPU based preform with injection techniques. This is mainly due to the design complexity limitation induced by injection moulding compared to the 3D printing. Known drilling, extrusion and injection moulding or mechanical tuning of TPU based preform would be difficult and time-consuming. 3D printing of the TPU-based preform has a huge competitive advantage for realizing waveguides that have a TPU-based cladding, with possibly a TPU based core.

Figures 25-27 show cross sections of complex shaped preforms that may be realized by 3D printing a TPU preform 200, 204. Such preforms 200, 204 may also be made in a TPE polymer.

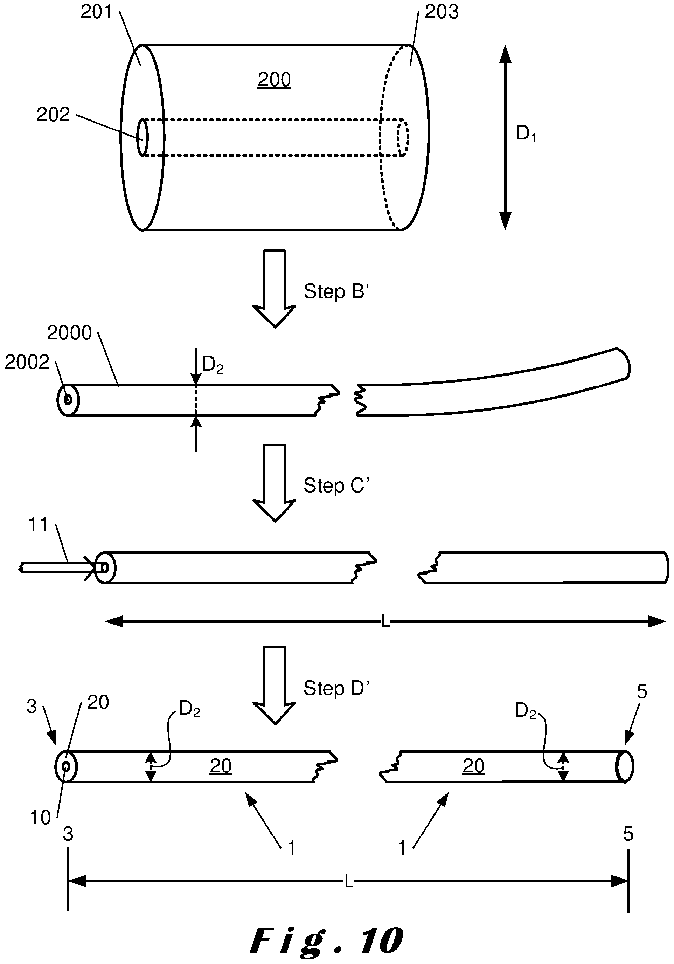

In a variant, a hollow shaped preform 204 is first formed and then an elongated capillary is realized that is filled with a polymer that may be different than a TPE elastomer, such as silicone. Such a method variant comprises the steps (A-D') that are illustrated schematically in Fig. 10:

A) realizing said hollow preform 204 made at least partially of a TPE elastomer as described above;

B') reducing the diameter of said hollow shaped preform 204 and elongating said preform until a capillary 2000 is formed having a predetermined length L and a predetermined cross section 40, 41, said capillary 2000 having a central opening 2002 having a predetermined cross section 30;

C') introducing liquid silicone 11 into the central opening 220 of said preform;

D') polymerising said liquid silicone so as to form an optical waveguide 1 having a core 10 being made of polymerised liquid silicone 11 and having a predetermined length L and an outside diameter D2. The diameter of the core is directly related to the proportion of the outside diameter of the preform D1 and the diameter of the aperture of the preform, because this proportion does not change during the diameter reduction step B';

In an embodiment step C' and D' are replaced by the steps E', F', G':

E') introducing liquid silicone 11 during said step B of reducing the diameter of said preform;

F') while reducing the diameter of said preform 200 keeping said liquid silicone in a liquid state until a predetermined length (L) and a predetermined cross section 40, 41 of a precursor optical waveguide is obtained;

G') thermal polymerising said liquid silicone 11 and said capillary 2000 so as to form an optical waveguide 1. In variants the capillary 2000 may be polymerised before the polymerisation of the liquid silicone;

In an embodiment steps B', C' and D' are replaced by the steps H, I, J:

H') after said step A, introducing a liquid polymer 11 into the central aperture 202' of said hollow preform 204;

I') reducing the diameter of the preform 204 filled with liquid polymer 11 and elongating said filled preform 200 until a capillary 2000 is formed filled with liquid polymer 11, said capillary having a predetermined length L and a predetermined cross section 40, 41;

J) polymerising said liquid polymer by applying UV light.

In an embodiment step said liquid polymer is liquid silicone, possibly a liquid siloxane.

In an embodiment of the method said obtained optical waveguide 1 is a multimode optical fiber. In a variant, said obtained waveguide is a mono-mode optical fiber.

In an embodiment said obtained waveguide is an optical waveguide having a non-circular cross section defined in any lateral plane X-Y. In embodiments the core 10 of the waveguide has a very small cross section, which may be smaller than 1 $\mu$m. A process to realize this is now described

In order to decrease the fiber diameter during the drawing, we will have to adjust the drawing parameters as the winding rate that will reduce the fiber diameter. In order to do so, both materials, TPU and silicone must be able to be processed at such small geometries. For silicone, that will be quite easy as it will remain liquid and will follow the TPU deformations. If the final fiber diameters are not small enough, it will be possible to start a new drawing process, if silicone has not yet polymerized, in order to reduce the fiber size. This process would be similar as the one proposed in Ref. 16 wherein an introduced fiber is melted while a rotating rod coils the microfiber. In our case the melting would be performed by the same heaters that are used to draw the fiber from the preform. Due to the small size of the incoming fiber, not greater than 100 $\mu$m, the melting should be achieved rapidly, and the silicone polymerization won't have the time again to happen. Coiling around a rod is mandatory rather than around a normal spool. The reason is that warm TPU fibers will get stuck to each other if they are not cooled enough. For a normal optical fiber drawing, this cooling is performed by taking away the furnace and the winding spool.

In an embodiment a length of useful optical waveguide 1 is determined by cutting it until an acceptable optical transmission is obtained. So, a transmission measurement step may be performed wherein the optical transmission ratio T1/T0 of said short optical waveguide is determined followed by a new step F consisting in cutting another predetermined length of said optical waveguide 1, so as to provide a second short optical waveguide having a length smaller than the length of said first short optical waveguide, said second short optical waveguide having a higher transmission ratio T2/T1 than the transmission ratio T1/T0 of said first short optical waveguide.

In variants, micro or nano-sized optical fibers may be realized. Their diameters may be typically 1 $\mu$m, possibly less than 1 $\mu$m. In case a preform made of TPE, such as TPU, is filled with UV polymerizable glue or silicone, the liquid that is introduced in the central aperture will follow the deformation of the TPU cylinder during its reduction of diameter so that the central aperture 2002 is not closed during the capillary pulling operation even when micro-sized diameters are reached. Once the predetermined length and outer diameter is reached the internal liquid is polymerized by UV light, through said TPE or TPU.

A Fan-IN/OUT optical system (Ref.17), allowing to connect the outputs and inputs of a multicore fiber (Ref.17) may be realized by a method comprising preferably the steps of:

- providing a plurality of optical fibers realized by the method of fabrication of the invention, having a non-cured liquid core and having a diameter typically of 40 $\mu$m;
- aligning the plurality of fibers in a mechanical holder, to form a bundle, having a plurality of holes and the same fibers geometry distribution as the multicore fiber. The mechanical holder has preferably a cylindrical external diameter that is equal or greater than the multicore fiber to connect;
- in an embodiment, the number of fibers introduced in the holder is 7. and may be different types of fiber;

- gluing the assembly of the mechanical holder, preferably by a UV glue, in order to fix all components together.

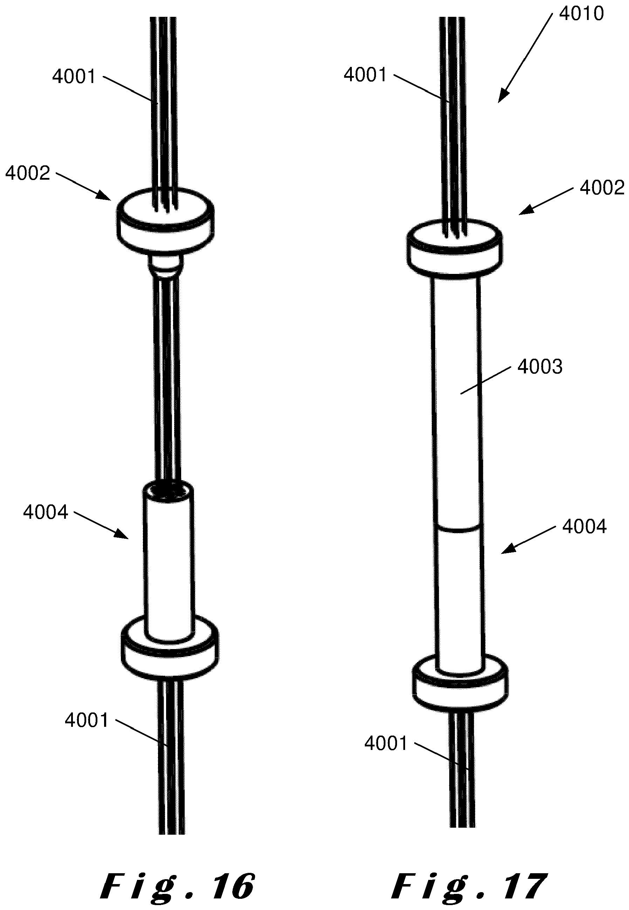

In an embodiment said plurality of optical fibers have a core made at least partially of TPE. In a variant, illustrated in Fig. 17, the preform is a hybrid preform. For example, a block 4000 may comprise two holders in which wires are arranged (as illustrated in Fig. 16) and this structure may be over moulded by a TPE, such as a TPU cladding layer as illustrated in Fig. 17. The preform 200 may be produced by injection moulding and the first half of the preform constitutes a multicore preform. The second half is a plurality of preferably 7 independent tubes directly aligned and connected to the first part of the preform comprising said two holders.

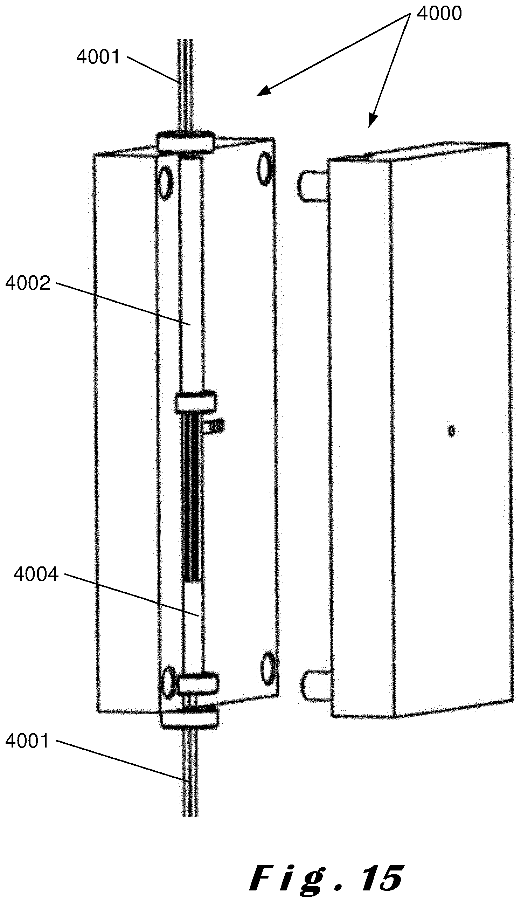

Figure 15 shows an example of an arrangement 4000 comprising a portion of a preform comprising two holders 4004, 4004, arranged preferably to realize a fan-in/fan-out optical component.

Figure 16 shows a portion of a preform comprising two holders of Fig.15, to make a fan-in/fan-out optical component, the two holders comprise a plurality of wires 4001 to be removed after injection of a TPE polymer Figure 16 illustrates two inserts comprising 7 mold cores before injection of the cladding. After overmoulding the volume between the two holders 4002, 4004 and so the wires present in that volume (Fig. 16) by a TPE layer 4003. The TPE is then cooled and the wires 4001 are withdrawn, leaving a plurality of axial holes in which another polymer, such as silicone may be introduced. The so formed preform may be drawn to make either a multicore fiber or may be drawn or heated over its central part so as to form a multi-core structure having a central portion with a reduced diameter, which may be used in a Fan-in/Fan-out optical device.

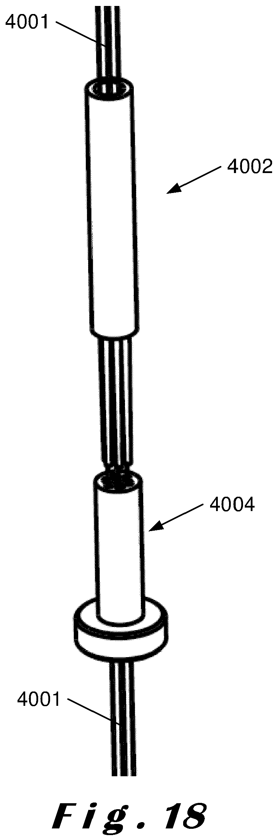

Figure 18 illustrates the demolding process of such a multicore fiber after the injection of a TPU layer 4003 as cladding layer illustrated in Fig. 17. Figure 19 illustrates a multicore Fan-in/Fan-out platform realized with the mold of Figure 16 and Fig. 17, and after melting and drawing to reach the final desired diameters. The fan-in/fan-out component 4100 illustrated in Fig. 19 comprises two ends 4104, 4106 having spaced cores and a middle section 4102 wherein the cores are closer than at the two ends 4104 and 4106.

The melt and draw process is realized in the transition area of the hybrid preform in order to decrease the size of the multicore preform part to a normal fiber size and a lower to the normal size for the tubes outgoing from the transition area. In an embodiment the fibers may be arranged in a ring or tube forming a multicore fiber structure-like. This structure is fixed by a thermal flash and then thermally drawn to reach a final diameter equal to the multicore fiber to be connected to.

An illumination device and method of illumination may be based on the use of varying diameter of the core of a fiber realized according to the method of fabrication of the invention. Such an illumination method comprises the steps of:

- providing an optical waveguide 1 realized according to the method of fabrication of the invention ;
- introducing light into the core 10 of the optical waveguide 1;
- stretching portions of the optical waveguide 1 so as to couple light out of the core 10 to its side and through said cladding 20.

In a variant, the stretching may be made periodically stretched by an automated mechanism. In an embodiment, dopants may be integrated in the TPE cladding layer to provide light diffusion effects by the cladding layer. By integrating dopants, it is possible to make more visible stretched portions of the optical waveguide and so provide lighting effects, useful in for example light decorations.

**Experimental results**

a. Realisation of a fiber with a TPU core and TPU cladding

In an exemplary process according to the invention three types of step-index optical fibers with the dimensions listed in Table 1 have been realized. For each fiber its V number has been determined, at three different wavelengths (633 nm, 830 nm and 1300 nm by considering the smallest diameter achieved, and the corresponding estimate of number of modes, by using the equation 1, and a numerical aperture (NA) of 0.38. It was assumed that the NA remains stable with wavelength, e.g., the materials are dispersion free. It may be noticed that the F3 fiber is approaching a few mode fiber (FMF) behaviour for wavelengths > 800 nm. The number of modes M is given by equation (1) and depends on the V number which is a dimensionless parameter which is often used in the context of step-index fibers. It is defined as

$$V = \frac{2\pi}{\lambda} a\, \mathrm{NA} = \frac{2\pi}{\lambda} a \sqrt{n_{core}^2 - n_{cladding}^2}$$

where $\lambda$ is the vacuum wavelength, a is the radius of the fiber core, and NA is the numerical aperture.

$$M = 4\frac{V^2}{\pi^2} \hspace{4cm} (1)$$

**Table 1.** Optical fibers produced

| Fiber name | Minimal core diameter | Cladding diameter | V number at λ [nm] | | | Estimated number of modes at λ [nm] | | |
|---|---|---|---|---|---|---|---|---|
| | | | 633 | 830 | 1300 | 633 | 830 | 1300 |
| F1 | 44 | 199 | 83.66 | 63.81 | 40.73 | 2'837 | 1'650 | 672 |
| F2 | 10 | 114 | 19.01 | 14.50 | 9.26 | 147 | 85 | 35 |
| F3 | 5.4 | 85 | 10.27 | 7.83 | 4.99 | 43 | 25 | 10 |

Realized fibers 1 according to the process of the invention have first been visually evaluated under a microscope and white light has been incoupled and guided in fiber lengths of 40 cm. Afterwards, the fibers attenuation was measured using the cut-back technique. Attenuation values obtained, listed in Table 2, are quite similar to those measured for TPU polymer flat sheets except for a 1300 nm wavelength. Attenuation was not measured for the third optical window (e.g. 1550 nm) because of the strong attenuation observed at this wavelength band.

**Table 2. Optical attenuation of realized TPU fibers produced**

| Fiber name | Attenuation at 633 nm [dB/cm] | Attenuation at 830 nm [dB/cm] | Attenuation at 1300 nm [dB/cm] |
|---|---|---|---|
| F1, F2 and F3 | 0.15-0.2 | 0.1-0.2 | 0.7-0.8 |

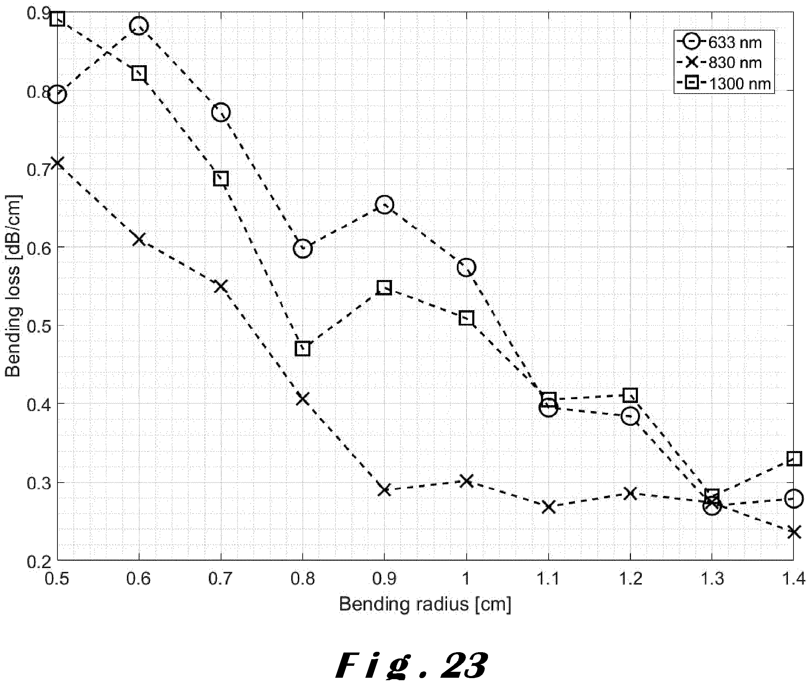

A set of sample fibers F3 was used to measure the bending loss and results are shown in Fig. 23. The fiber F3 was bent on metallic rods with two turns and manually maintained while the power signal was stabilizing. The TPU fibers are so soft that any touch makes a direct stress on the core-cladding interface and thus a variation in the optical transmission when fiber is bent. Additionally, to bend the fiber around a rod, there is inevitably a non-negligible elongation of the fiber which induce an additional loss. However, the fibers may be well used for short lengths in applications where small bend radius are needed.

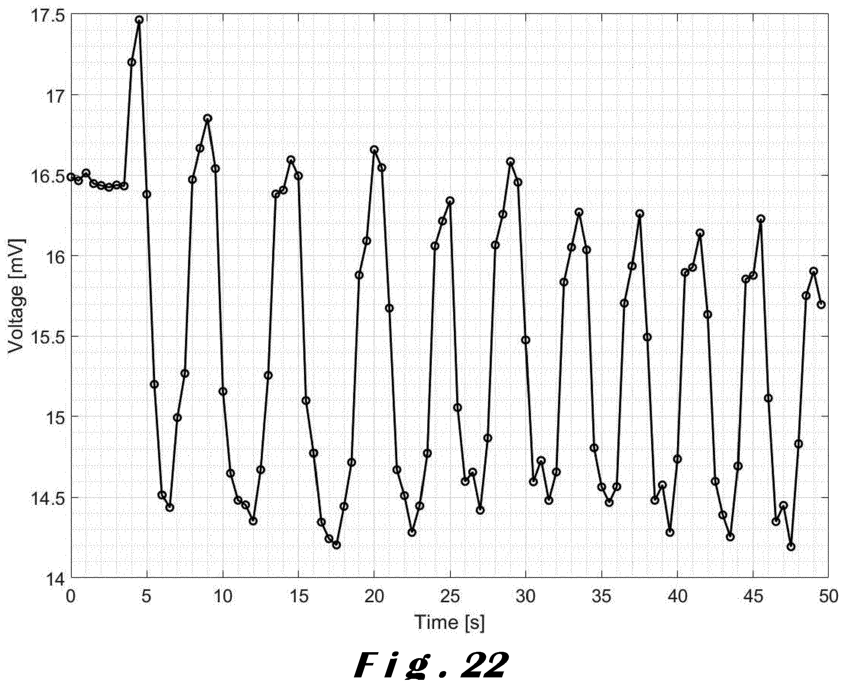

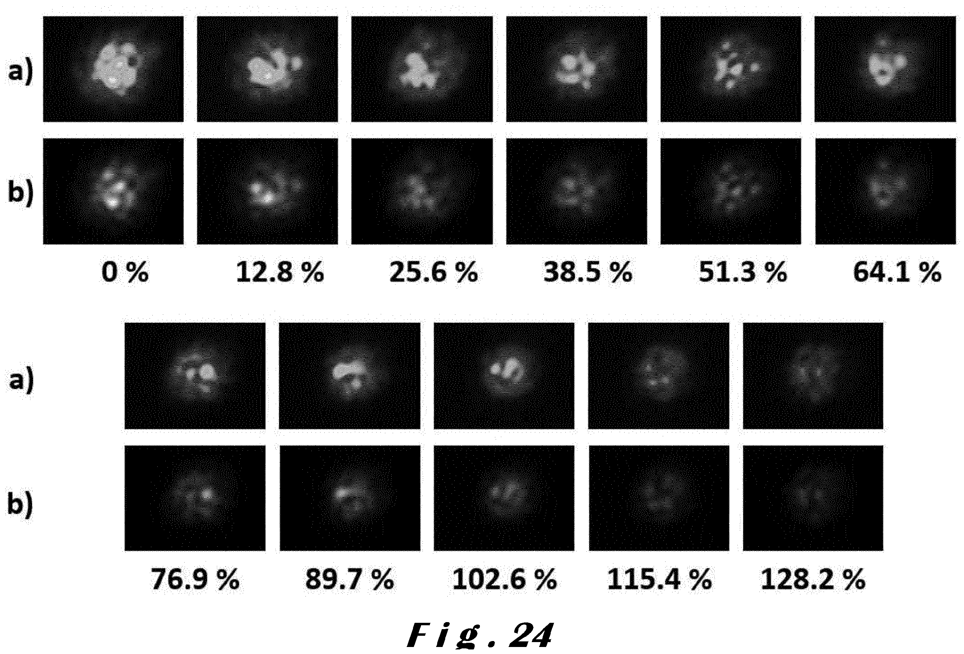

The variation of transmitted intensities of the optical waveguide 1 can be used to realize a pressure sensor or a system in which the transmitted intensity varies according to applied pressure or bending. Fig.22 shows experimental results of variations of intensity b using a 8cm length of a fiber made of a TPU core and TPU cladding by adhering the fiber to the outside surface of an inflatable balloon. Fig.22 shows the variations in detected intensities by inflating the balloon. The fiber underwent a variation in length of up to 30%. The dimension of the core changed by 30%, from initially $10\mu m$ to $6.6\mu m$. The wavelength used was 633nm. Fig.24 illustrates the change of the number of modes in function of the elongations that the fiber underwent and are expressed in %. The initial length is illustrated by 0% and 128% means the length has increased by a factor of 2.28.

b. Realisation of a fiber with a TPU cladding and a silicone core

In an experimental process according to the invention, a TPU based capillary is filled with silicone to realize an optical fiber. In such process of a TPU capillary 2000, the first step is to produce a cladding preform 200, preferably by injection moulding. The cladding dimensions has to respect the ratio of the final cladding diameter and core fiber diameter in order to obtain a multimode fiber having a predetermined number of guides modes, or to ensure a fiber and core size that may be close to or equal to standard single mode fibers. The cladding diameter must comply with the moulding dimension constrains but also depends on the initial central hole diameter that is needed to use an insert inside the mould. This insert is a critical component of the cladding production as it can be crooked by the hot polymer flow during the moulding process. Based on these constraints and typical injection moulding equipment, typical cladding diameters are up to 20 mm for a length of about 100 mm. The central hole diameter of the preform 200 is normally not less than 1 mm because of the polymer flow stress during moulding. Hole diameters of the preform as low as 0.5 mm may be obtained. Another constraint on the insert is its surface quality and adherence to the TPU. The surface quality will directly influence the optical interface between TPU and silicone, and fortiori, the optical guidance losses inside the final fiber. Thus, a surface treatment is in principle needed in order to reduce the roughness and the TPU adherence.

Once the cladding preform is obtained, a standard process of thermal fiber drawing provides a hair thin TPU fiber capillary. Because the lengths of fiber that are needed for most applications addressed here, are quite short (10-15 cm), the conicity and diameter fluctuations are less constricting and hence relax the process constraints.

Tests have been performed to realize silicone penetration in small diameter capillaries, glass capillaries 2000 are used having a central hole 2002 of 15 $\mu$m and external diameter of 125 $\mu$m. By using the syringe technique, it is possible to inject silicone in a length of around 100 mm. By using vacuum pump techniques together with a syringe, it is possible to achieve a penetration depth of more than 100 mm. By modifying the wettability of the inner capillary walls, hence with the help of the capillary filling force, the initial penetration length is improved. In addition, by using more performant vacuum pumps, and by increasing the syringe efficiency on the other side of the fiber, penetrations depths over fiber lengths up to 20-40 cm are achievable.

In a typical silicone-based optical fiber the core material is made of a silicone that is usually used as a LED liquid encapsulant. The silicone is an OPTOLINQ trademark OLS-5291-type silicone commercialized by Caplinq Corporation (Canada).

The cladding material was a TPU polymer that can be found at very soft grades, such as the BASF 1185A TPU. Achieved cladding dimension may be 200$\mu$m and the core diameter 50$\mu$m and the length of the fiber 1 may be at least 250mm.The fiber has a typical a high numerical aperture of 0.32.Typical realized fibers may be stretched by about 50%, i.e. elongating the optical fiber up to 375mm without notable optical losses. Bending losses were less than 20-30% due to the high numerical aperture of the fiber1. This cannot be achieved by other polymer-based fibers of prior art such as PMMA fibers. Tests have shown that squeezing an optical fiber 1 realized by the method of fabrication of the invention does not alter its mechanical or optical properties. For example, by applying a lateral force of 20N -30N the optical fiber returns to its initial shape without any changes of its mechanical or optical properties.

Typical optical transmissions of the optical waveguide 1, measured by an optical bench are:

- at 633 nm the attenuation was 0.2 - 0.3 dB/cm;
- at 1300 nm the attenuation was: 0.3 - 0.5 dB/cm;
- at 1550 nm a typical attenuation was 0.6 - 0.9 dB/cm.

The optical waveguide 1 realized by the method of fabrication of the invention may be used for some UV applications, if the lengths are typically shorter than 100mm. In the UV , estimates of transmission were about 30-60% at 300nm for a length of 50mm, but the transmission value may vary considerably according to the type of polymers that are used and of course of the UV wavelength, Transmissions below 300nm are typically lower than 20-10% for lengths of fiber of about 50mm.

**Applications of the waveguide 1 realized by the method of fabrication of the invention**

One of the important applications of the optical waveguide realized by the method of the invention 1 relates to implants and more specifically cochlear implants. For such applications the 3D printing of the TPU-based preform as described herein is particularly well adapted. The optical waveguide 1 has been implemented in a pressure sensor tip arranged at a cochlear implant tip. The optical waveguide 1 is intended to minimize accidental structural intracochlear damage. Recent studies have demonstrated pressure pulses equivalent to sound levels causing severe impulse trauma during implantation, caused by the insertion of the electrode array into the enclosed space of the cochlea. A solution implementing the optical waveguide 1 realized by the method of the invention will improve surgery reliability and flexibility and also the implant quality by avoiding any failure during the critical process of implantation. A cochlear implant cost, including surgery, can vary between USD 30'000 - 50'000. A failed surgery, though rare, will induce a second surgery preceded by a patient recovery time whilst an implant dysfunction (due to structural damage) is potentially possible as well. Additionally, the residual structures and neural tissue in the cochlea are highly relevant to the sound quality experienced by the patient. The insertion process can be highly traumatic to these structures, due to pressure pulses caused by surgeon handling and penetration of important membranes. Novel optical fiber technologies may revolutionize the surgical procedure, reducing fibrotic tissue growth and also maintaining the cochlear condition for successful future regenerative therapies to enhance the outcome. Furthermore, implantable optical technology could also improve the post-operative rehabilitation by continuously monitoring physiological parameters. The optical waveguide realized by the method of the invention allows to decrease dramatically implants failures as well as surgery failures by providing a feedback measurement of important physiological or environmental parameters. Furthermore, it opens the door for long term and highly localized monitoring of physiological parameters. This is of high interest as it will enable to make an early detection of medical issues, enabling in this way to minimize possible post-implantation traumas and their consequent surgeries. It is also providing a solution to diminish wastes by increasing the reliability of implants and the consumables needed for surgeries. Subsequently, the potential diminution of wastes is obviously enhancing the energy efficiency of the implantation activities by reducing the implantation failures and their possible traumas but as well by preventing important surgeries on a long-term point of view.

Other important applications that may profit from the optical waveguide 1 realized by the method of the invention are, but not exclusively:

- ophthalmology: a pressure sensing device using the optical waveguide 1 is useful in cataract surgery;
- spinal traumatology: Pressure monitoring after surgery (vertebra or disc replacement);
- heart traumatology: blood flow and pressure monitoring after a heart attack, localized micro-surgery;
- cardiac surgery, orthopedics, urology, and neurology;
- security and counterfeit applications;
- industrial sensor applications.

As a totally new class of optical waveguides, different applications of the waveguide realized by the method of the invention could generate new markets. As an example, Bragg gratings may be provided on the TPU-based waveguide 1 to improve its sensitivity with no need of any additional sensor. The optical waveguide 1 realized by the method of the invention can be used for sensing, as explained for implants manufacturers, but could also be used to bring light at remote locations where glass optical fibers would be hazardous to use. The optical waveguides 1 realized by the method of the invention may also be used in FAN/IN-FAN/OUT systems, such as for example described in Ref.15.

Still other applications address decorative illumination devices. A TPE such as TPU cladding may be doped with diffusing particles. In an example, light may be made more or less visible in sections where the core has a reduced size by pulling a section of the optical waveguide. In other applications Bragg gratings may be arranged on the optical waveguide 1 realized by the method of the invention and allow to replace for example SiO2-based optical waveguides or fibers. Such waveguides 1 may be used for cryogenic applications where the thermal dilation of SiO2 is substantially non existing.

## Back-ground information and References

[1] Gerd Keiser, Fei Xiong, Ying Cui and Perry Ping Shum, "Review of diverse optical fibers used in biomedical research and clinical practice", Journal of Biomedical Optics 19(8), 080902 (August 2014)

[2] Sedat Nizamoglu, Malte C. Gather, and Seok Hyun Yun, "All-Biomaterial Laser Using Vitamin and Biopolymers", Adv. Mater. 2013, 25, 5943-5947

[3] M. Llera, T. Aellen, J. Hervas, Y. Salvadé, P. Senn, S. Le Floch and H. Keppner, "Liquid-air based Fabry-Pérot cavity on fiber tip sensor", Opt. Express 24, 8054-8065 (2016)

[4] Sara T. Parker, and al., "Biocompatible Silk Printed Optical Waveguides", Adv. Mater.2009,21,2411-2415

[5] Myunghwan Choi, Matjaž Humar, Seonghoon Kim, and Seok-Hyun Yun, "Step-Index Optical Fiber Made of Biocompatible Hydrogels", Adv. Mater. 2015, 27, 4081-4086;

[6] M. Han, and A. Wang, "Exact analysis of low-finesse multimode fiber extrinsic Fabry-Perot interferometers", Applied Optics (43), pp. 4659-4666, 2004;

[7] Brandon W. Swatowski, Chad M. Amb, W. Ken Weidner, Ranjith S. John, Jeffrey D. Mitchell, "Advances in manufacturing of optical silicone waveguides for high performance computing" 2014 IEEE Avionics, Fiber-Optics and Photonics Technology Conference (AVFOP), ThB1, 2014

[8] Md Rejvi Kaysir, Alessio Stefani, Richard Lwin, and Simon Fleming, "Flexible optical fiber sensor based on polyurethane", 2017 Conference on Lasers and Electro-Optics Pacific Rim (CLEO-PR), 2017

[9] Timo Stöver and Thomas Lenarz, "Biomaterials in cochlear implants", GMS Curr Top Otorhinolaryngol Head Neck Surg, 2009

[10] http://info.hotims.com/45604-161

[11] Hugh L. Narcisco, Jr., "Silicone optical waveguide", US5237638A, 1991

[12] Nathan N. Haese, and al. "Pressure sensor utilizing a polyurethane optical fiber", US4915473A, 1989

[13] https://www.aliexpress.com/

[14] Hervé Elettro. Elastocapillary windlass: from spider silk to smart actuators. Mechanics of the fluids [physics.class-ph]. UPMC, 2015.

[15] O.Shimakawa et al. Connector type fan-out device for multi-core fiber ", SEI Tech Review nr 77, pp.23-28, Oct.2013.

[16] [M. Sumetsky, Y. Dulashko, S. Ghalmi, "Fabrication of miniature optical fiber and microfiber coils", Opt. Lasers Eng., 48 (2010), pp. 272-275].

[17] O. Shimakawa, H. Arao, M. Shiozaki, T. Sano, A. Inoue, "Connector type fan-out device for multi-core fiber", SEI Tech. Review, no. 77, pp. 23-28, Oct. 2013.

[18] S.Chen et al., "Near-infrared deep brain stimulation via upconversion nano-particle-mediated optogenetics", Science 359, 2088, pp.679-684, Feb. 2018.

**Claims**

1. A method of fabrication of an optical waveguide (1) being configured to transmit along a longitudinal axis a light beam (100) having a wavelength greater than 180nm, comprising the steps, A-C, of:

   A. Realizing a hollow shaped preform (204) which will form the cladding (20) of the optical waveguide (1), the hollow shaped preform (204) comprising an innermost layer made at least partially of a thermoplastic elastomer, TPE, having a second refractive index, n2, and a central aperture (202');
   B. Filling the central aperture (202') of said hollow shaped preform (204) so as to produce the core part of the preform (200) which will form the core of the optical waveguide (1), said core part being made of a thermoplastic elastomer (TPE) or of silicone or of siloxane, comprising an outermost layer and having a first index of refraction n1, being higher than said second index of refraction n2, said innermost layer being formed in contact with said outermost layer, so as to provide a filled preform (200);
   C. reducing the diameter of said filled preform (200) and elongating said filled preform (200) to obtain an optical waveguide (1) having a predetermined length L and a predetermined cross section (40, 41).

2. The method according to claim 1 wherein, in said step B, the central aperture (202') of the hollow shaped preform (204) is filled with a thermoplastic elastomer TPE, chosen among a thermoplastic polyurethane TPU, a styrenic block copolymer, TPE-s, a thermoplastic polyolefin elastomer, TPE-o, a thermoplastic Vulcanizate, TPE-v, TPV, a thermoplastic copolyester , TPE-E, a thermoplastic polyamide, TPE-A, or a not classified thermoplastic elastomer, TPZ.

3. A method of fabrication of an optical waveguide (1) being configured to transmit along a longitudinal axis a light beam (100) having a wavelength greater than 180nm, having a core made of silicone or siloxane comprising the steps , A, B'-D', of:

   A) Realizing a hollow shaped preform (204) which will form the cladding (20) of the optical waveguide (1), the hollow shaped preform (204) comprising an innermost layer made at least partially of a thermoplastic elastomer, TPE, having a second refractive index, n2, and a central aperture (202');
   B') reducing the diameter of said hollow shaped preform (204) and elongating it until a capillary (2000) is formed having a predetermined length , L, and a predetermined cross section (40, 41), said capillary (2000) having a central aperture (2002) having a predetermined cross section (30);
   C') introducing liquid silicone or siloxane (11) into the central aperture (2002) of said capillary (2000);
   D') polymerising said liquid silicone or siloxane (11) so as to form an optical waveguide (1) having a core (10) being made of polymerised liquid silicone or siloxane (11).

4. A method of fabrication of an optical waveguide (1) being configured to transmit along a longitudinal axis a light beam (100) having a wavelength greater than 180nm, having a core made of silicone or siloxane, comprising the steps, A, B', E', F', G',

A) realizing a hollow shaped preform (204) which will form the cladding (20) of the optical waveguide (1), the hollow shaped preform (204) comprising an innermost layer made at least partially of a thermoplastic elastomer , TPE, having a second refractive index, n2, and a central aperture (202');

B') reducing the diameter of said hollow shaped preform (204) and elongating it until a capillary (2000) is formed having a predetermined length, L, and a predetermined cross section (40, 41), said capillary (2000) having a central aperture (2002) having a predetermined cross section (30);

E') introducing liquid silicone or siloxane (11) in the central aperture (202') during said step B' of reducing the diameter of said hollow shaped preform (204);

F') while reducing the diameter of said hollow shaped preform (204) keeping said liquid silicone or siloxane in a liquid state until a predetermined length, L, and a predetermined cross section (40, 41) of a precursor optical waveguide is obtained;

G') thermal polymerising said liquid silicone or siloxane (11) and said capillary (2000) so as to form an optical waveguide (1).

**5.** The method according to any one of claims 1 to 4 wherein said preform (200) is made in TPU by a 3D printing technique.

**Patentansprüche**

**1.** Verfahren zur Herstellung eines Lichtwellenleiters (1), der dafür gestaltet ist, einen Lichtstrahl (100) mit einer Wellenlänge größer als 180nm entlang einer Längsachse zu übertragen, umfassend die folgenden Schritte A bis C:

A. Realisieren einer hohlen Preform (204), die den Mantel (20) des Lichtwellenleiters (1) bilden wird, wobei die hohle Preform (204) eine zumindest teilweise aus einem thermoplastischen Elastomer, TPE, bestehende innerste Schicht mit einem zweiten Brechungsindex, n2, und einen mittigen Durchlass (202') umfasst;

B. Füllen des mittigen Durchlasses (202') der hohlen Preform (204), um den Kernteil der Preform (200) herzustellen, der den Kern des Lichtwellenleiters (1) bilden wird, wobei der Kernteil aus einem thermoplastischen Elastomer (TPE) oder aus Silikon oder aus Siloxan besteht, eine äußerste Schicht umfasst und einen ersten Brechungsindex, n1, aufweist, der höher als der zweite Brechungsindex, n2, ist, wobei die innerste Schicht in Kontakt mit der äußersten Schicht ausgebildet ist, um eine gefüllte Preform (200) bereitzustellen;

C. Reduzieren des Durchmessers der gefüllten Preform (200) und Ausziehen der gefüllten Preform (200), um einen Lichtwellenleiter (1) mit einer vorgegebenen Länge L und einem vorgegebenen Querschnitt (40, 41) zu erhalten.

**2.** Verfahren nach Anspruch 1, wobei im Schritt B der mittige Durchlass (202') der hohlen Preform (204) mit einem thermoplastischen Elastomer, TPE, gefüllt wird, das aus einem thermoplastischen Polyurethan, TPU, einem Styrol-Blockcopolymer, TPE-S, einem thermoplastischen Polyolefin-Elastomer, TPE-O, einem thermoplastischen Vulkanisat, TPE-V, TPV, einem thermoplastischen Copolyester, TPE-E, einem thermoplastischen Polyamid, TPE-A, oder einem nicht klassifizierten thermoplastischen Elastomer, TPZ, ausgewählt ist.

**3.** Verfahren zur Herstellung eines Lichtwellenleiters (1), der dafür gestaltet ist, einen Lichtstrahl (100) mit einer Wellenlänge größer als 180nm entlang einer Längsachse zu übertragen, und einen aus Silikon oder Siloxan bestehenden Kern aufweist, umfassend die folgenden Schritte A, B' bis D' :

A) Realisieren einer hohlen Preform (204), die den Mantel (20) des Lichtwellenleiters (1) bilden wird, wobei die hohle Preform (204) eine zumindest teilweise aus einem thermoplastischen Elastomer, TPE, bestehende innerste Schicht mit einem zweiten Brechungsindex, n2, und einen mittigen Durchlass (202') umfasst;

B') Reduzieren des Durchmessers der hohlen Preform (204) und Ausziehen derselben, bis eine Kapillare (2000) mit einer vorgegebenen Länge, L, und einem vorgegebenen Querschnitt (40, 41) ausgebildet ist, wobei die Kapillare (2000) einen mittigen Durchlass (2002) mit einem vorgegebenen Querschnitt (30) aufweist;

C') Einleiten von flüssigem Silikon oder Siloxan (11) in den mittigen Durchlass (2002) der Kapillare (2000);

D') Polymerisieren des flüssigen Silikons oder Siloxans (11), um einen Lichtwellenleiter (1) mit einem aus polymerisiertem flüssigem Silikon oder Siloxan (11) bestehenden Kern (10) auszubilden.

**4.** Verfahren zur Herstellung eines Lichtwellenleiters (1), der dafür gestaltet ist, einen Lichtstrahl (100) mit einer Wellenlänge größer als 180nm entlang einer Längsachse zu übertragen, und einen aus Silikon oder Siloxan bestehenden Kern aufweist, umfassend die folgenden Schritte A, B', E', F' und G':

A) Realisieren einer hohlen Preform (204), die den Mantel (20) des Lichtwellenleiters (1) bilden wird, wobei die hohle Preform (204) eine zumindest teilweise aus einem thermoplastischen Elastomer, TPE, bestehende innerste Schicht mit einem zweiten Brechungsindex, n2, und einen mittigen Durchlass (202') umfasst;

B') Reduzieren des Durchmessers der hohlen Preform (204) und Ausziehen derselben, bis eine Kapillare (2000) mit einer vorgegebenen Länge, L, und einem vorgegebenen Querschnitt (40, 41) ausgebildet ist, wobei die Kapillare (2000) einen mittigen Durchlass (2002) mit einem vorgegebenen Querschnitt (30) aufweist;

E') Einleiten von flüssigem Silikon oder Siloxan (11) in den mittigen Durchlass (202') während des Schritts B' des Reduzierens des Durchmessers der hohlen Preform (204);

F') während des Reduzierens des Durchmessers der hohlen Preform (204) Halten des flüssigen Silikons oder Siloxans in einem flüssigen Zustand, bis eine vorgegebene Länge, L, und ein vorgegebener Querschnitt (40, 41) eines Vorläufer-Lichtwellenleiters erhalten werden;

G') thermisches Polymerisieren des flüssigen Silikons oder Siloxans (11) und der Kapillare (2000), um einen Lichtwellenleiter (1) auszubilden.

5. Verfahren nach einem der Ansprüche 1 bis 4, wobei die Preform (200) mit einer 3D-Drucktechnik aus TPU hergestellt wird.

**Revendications**

1. Procédé de fabrication d'un guide d'ondes optique (1) conçu pour transmettre, le long d'un axe longitudinal, un faisceau lumineux (100) présentant une longueur d'onde supérieure à 180 nm, comprenant les étapes A-C de :

A. réalisation d'une préforme façonnée creuse (204) qui formera la gaine (20) du guide d'ondes optique (1), la préforme façonnée creuse (204) comprenant une couche la plus interne au moins partiellement en un élastomère thermoplastique, TPE, présentant un second indice de réfraction, n2, et une ouverture centrale (202') ;

B. remplissage de l'ouverture centrale (202') de ladite préforme façonnée creuse (204) de manière à produire la partie coeur de la préforme (200) qui formera le coeur du guide d'ondes optique (1), ladite partie coeur étant en un élastomère thermoplastique (TPE) ou en silicone ou en siloxane, comprenant une couche la plus externe et présentant un premier indice de réfraction, n1, supérieur audit second indice de réfraction, n2, ladite couche la plus interne étant formée en contact avec ladite couche la plus externe, de manière à obtenir une préforme (200) remplie ;

C. réduction du diamètre de ladite préforme (200) remplie et allongement de ladite préforme (200) remplie afin d'obtenir un guide d'ondes optique (1) présentant une longueur prédéterminée L et une section transversale prédéterminée (40, 41).

2. Procédé selon la revendication 1, dans lequel, dans ladite étape B, l'ouverture centrale (202') de la préforme façonnée creuse (204) est remplie d'un élastomère thermoplastique, TPE, choisi parmi un polyuréthane thermoplastique, TPU, un copolymère séquencé styrénique, TPE-S, un élastomère de polyoléfine thermoplastique, TPE-O, un vulcanisat thermoplastique, TPE-V, TPV, un copolyester thermoplastique, TPE-E, un polyamide thermoplastique, TPE-A, ou un élastomère thermoplastique non classé, TPZ.

3. Procédé de fabrication d'un guide d'ondes optique (1) conçu pour transmettre, le long d'un axe longitudinal, un faisceau lumineux (100) présentant une longueur d'onde supérieure à 180 nm, présentant un coeur en silicone ou en siloxane, comprenant les étapes A, B'-D' de :

A) réalisation d'une préforme façonnée creuse (204) qui formera la gaine (20) du guide d'ondes optique (1), la préforme façonnée creuse (204) comprenant une couche la plus interne au moins partiellement en un élastomère thermoplastique, TPE, présentant un second indice de réfraction, n2, et une ouverture centrale (202') ;

B') réduction du diamètre de ladite préforme façonnée creuse (204) et allongement de cette dernière jusqu'à ce qu'un capillaire (2000) soit formé présentant une longueur prédéterminée, L, et une section transversale prédéterminée (40, 41), ledit capillaire (2000) présentant une ouverture centrale (2002) présentant une section transversale prédéterminée (30) ;

C') introduction de silicone ou de siloxane (11) liquide dans l'ouverture centrale (2002) dudit capillaire (2000) ;

D') polymérisation de ladite silicone ou dudit siloxane (11) liquide de manière à former un guide d'ondes optique (1) présentant un coeur (10) en silicone ou en siloxane (11) liquide polymérisé(e).

4. Procédé de fabrication d'un guide d'ondes optique (1) conçu pour transmettre, le long d'un axe longitudinal, un faisceau lumineux (100) présentant une longueur d'onde supérieure à 180 nm, présentant un coeur en silicone ou en siloxane, comprenant les étapes A, B', E' F', G' de :

A) réalisation d'une préforme façonnée creuse (204) qui formera la gaine (20) du guide d'ondes optique (1), la préforme façonnée creuse (204) comprenant une couche la plus interne au moins partiellement en un élastomère thermoplastique, TPE, présentant un second indice de réfraction, n2, et une ouverture centrale (202') ;

B') réduction du diamètre de ladite préforme façonnée creuse (204) et allongement de cette dernière jusqu'à ce qu'un capillaire (2000) soit formé présentant une longueur prédéterminée, L, et une section transversale prédéterminée (40, 41), ledit capillaire (2000) présentant une ouverture centrale (2002) présentant une section transversale prédéterminée (30) ;

E') introduction de silicone ou de siloxane (11) liquide dans l'ouverture centrale (202') lors de ladite étape B' de réduction du diamètre de ladite préforme façonnée creuse (204) ;

F') tout en réduisant le diamètre de ladite préforme façonnée creuse (204), maintien de ladite silicone ou dudit siloxane liquide à l'état liquide jusqu'à une longueur prédéterminée, L, et une section transversale prédéterminée (40, 41) d'un guide d'ondes optique précurseur soient obtenus ;

G') polymérisation thermique de ladite silicone ou dudit siloxane (11) liquide et dudit capillaire (2000) de manière à former un guide d'ondes optique (1).

5. Procédé selon l'une quelconque des revendications 1 à 4, ladite préforme (200) étant en TPU par une technique d'impression 3D.

*Fig.1*

*Fig.2*

*Fig.3*

*Fig.4*

**Fig. 5**

**Fig. 6**

**Fig. 7**

**Fig. 8**

**Fig. 9**

**Fig. 10**

*F i g . 11*

*F i g . 12*

EP 4 150 386 B1

*Fig. 13*

*Fig. 14*

EP 4 150 386 B1

4000

4001

4002

4004

4001

*F i g . 15*

**Fig. 16**

**Fig. 17**

4001

4002

4004

4001

*Fig . 18*

204    202'    204'

*Fig. 19*

20    10

0.1 mm

*Fig. 20*

*Fig. 21*

*Fig. 22*

*F i g . 23*

EP 4 150 386 B1

0 %    12.8 %    25.6 %    38.5 %    51.3 %    64.1 %

76.9 %    89.7 %    102.6 %    115.4 %    128.2 %

*F i g . 24*

EP 4 150 386 B1

Fig. 25

Fig. 26

Fig. 27

36

*Fig. 28*

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP S59111952 B
- WO 2003091178 A2
- CN 107589507
- CN 206431340
- US 2013243948 A
- CN 203275734
- US 4915473 A
- US 20080089088 A1
- US 4893897 A
- JP 62269905 B
- JP S5447667 B

- JP S6230152 B
- JP H01286939 B
- CN 108977069
- US 5237638 A
- US 5692088 A
- US 9700655 B2
- US 2012244143 A
- CN 107907484
- US 2016177002 A
- US 2016281267 A1
- US 4909597 A

### Non-patent literature cited in the description

- **ANDREAS LEBER et al.** ADVANCED FUNCTIONAL MATERIALS. WILEY-V CH VERLAG GMBH&CO, 07 December 2018, vol. 29
- **A.SHARON et al.** Resonating grating-waveguide structures for visible and near-infrared radiation. *J.Opt.Soc.Am,* 1997, vol. 14 (11), 2985-2993
- **GERD KEISER ; FEI XIONG ; YING CUI ; PERRY PING SHUM.** Review of diverse optical fibers used in biomedical research and clinical practice. *Journal of Biomedical Optics,* August 2014, vol. 19 (8), 080902
- **SEDAT NIZAMOGLU ; MALTE C. GATHER ; SEOK HYUN YUN.** All-Biomaterial Laser Using Vitamin and Biopolymers. *Adv. Mater.,* 2013, vol. 25, 5943-5947
- **M. LLERA ; T. AELLEN ; J. HERVAS ; Y. SALVADÉ ; P. SENN ; S. LE FLOCH ; H. KEPPNER.** Liquid-air based Fabry-Pérot cavity on fiber tip sensor. *Opt. Express,* 2016, vol. 24, 8054-8065
- **SARA T. PARKER.** Biocompatible Silk Printed Optical Waveguides. *Adv. Mater.,* 2009, vol. 21, 2411-2415
- **M. HAN ; A. WANG.** Exact analysis of low-finesse multimode fiber extrinsic Fabry-Perot interferometers. *Applied Optics,* 2004, vol. 43, 4659-4666

- **BRANDON W. SWATOWSKI ; CHAD M. AMB ; W. KEN WEIDNER ; RANJITH S. JOHN ; JEFFREY D. MITCHELL.** Advances in manufacturing of optical silicone waveguides for high performance computing. *2014 IEEE Avionics, Fiber-Optics and Photonics Technology Conference (AVFOP),* 2014
- **MD REJVI KAYSIR ; ALESSIO STEFANI ; RICHARD LWIN ; SIMON FLEMING.** Flexible optical fiber sensor based on polyurethane. *2017 Conference on Lasers and Electro-Optics Pacific Rim (CLEO-PR),* 2017
- **TIMO STÖVER ; THOMAS LENARZ.** Biomaterials in cochlear implants. *GMS Curr Top Otorhinolaryngol Head Neck Surg,* 2009
- **HERVÉ ELETTRO.** Elastocapillary windlass: from spider silk to smart actuators. *Mechanics of the fluids [physics.class-ph,* 2015
- **O.SHIMAKAWA et al.** Connector type fan-out device for multi-core fiber. *SEI Tech Review,* October 2013, (77), 23-28
- **M. SUMETSKY ; Y. DULASHKO ; S. GHALMI.** Fabrication of miniature optical fiber and microfiber coils. *Opt. Lasers Eng.,* 2010, vol. 48, 272-275
- **O. SHIMAKAWA ; H. ARAO ; M. SHIOZAKI ; T. SANO ; A. INOUE.** Connector type fan-out device for multi-core fibe. *SEI Tech. Review,* October 2013, (77), 23-28
- **S.CHEN et al.** Near-infrared deep brain stimulation via upconversion nano-particle-mediated optogenetics. *Science,* February 2018, vol. 359 (2088), 679-684